# EUROPEAN PATENT APPLICATION

(11) **EP 4 843 577 A1**
(43) Date of publication of application: **25.11.2026**
(21) Application number: 25741582.8
(22) Date of filing: 16.01.2025
(51) Int. Cl.: A61K 31/7088, A61K 48/00, C07K 17/00, A61P 21/00

(54) **POLYPEPTIDE-OLIGONUCLEOTIDE COMPLEX AND USE THEREOF**

(30) Priority: 17.01.2024 CN 202410071560
(71) Applicant: Assembly Medicine, LLC., Shanghai, 201203 (CN)
(72) Inventor: ZHOU, Liujuan, Shanghai 201203 (CN); BAI, Zhaoshuai, Shanghai 201203 (CN); PAN, Liqiang, Shanghai 201203 (CN); CHOU, James Jeiwen, Shanghai 201203 (CN)
(74) Representative: Lavoix
(86) International application number: PCT/CN2025/072756
(87) International publication number: WO 2025/153021

(57) **Abstract**

Provided are a polypeptide-oligonucleotide polymer complex and a use thereof. Specifically, provided is a polymer complex carrying at least one (al) polypeptide element and at least one (bl) oligonucleotide pharmacodynamic element, which are linked by means of a complementary nucleic acid backbone composite structure.

## Description

### TECHNICAL FIELD

The present invention relates to the field of biopharmaceuticals, specifically to polypeptide-oligonucleotide complex and a use thereof.

### BACKGROUND

Due to the potential of oligonucleotides to precisely regulate gene expression for the treatment of diseases, oligonucleotide-based gene therapy has become increasingly popular. Oligonucleotides generally range from 20 to 60 base pairs (bps) in length and can exist in either single-stranded or double-stranded forms, and nucleic acid monomers include ribonucleotides, deoxyribonucleotides, and other nucleic acid derivatives. Oligonucleotide drugs currently under development include antisense oligonucleotide (AON), small interfering RNA (siRNA), microRNA (miRNA), and nucleic acid aptamer. Oligonucleotide drugs pair with DNA, mRNA, or pre-mRNA through the principle of complementary base pairing to achieve precise regulation of gene expression. They have further expanded the means and boundaries of disease treatment, demonstrating significant potential for drug development.

However, oligonucleotide drugs also face certain challenges, including difficulty in crossing cell membranes and the blood-brain barrier, as well as a lack of targeting capability. Consequently, antibody-oligonucleotide conjugate (AOC), which possesses targeting capabilities, have emerged. An AOC is a novel biomacromolecule formed by the conjugation of an antibody and an oligonucleotide. Oligonucleotides have the ability to regulate gene expression and are being developed as gene therapy drugs, while the conjugated antibody provides the oligonucleotide with targeting capabilities, offering more precise targeting and better pharmacokinetic properties compared to oligonucleotides alone.

AOC candidates are primarily in the preclinical and early clinical stages, with no AOC drugs currently on the market. Indications for AOC candidates under development include muscular disorders, central nervous system disorders, and cancer, etc. Because the concept of AOCs is relatively new and the preparation process is complex and requires stringent conditions, only a handful of companies currently possess AOC technology platforms, and the technical barriers are high.

Therefore, there is an urgent need to develop novel AOC drugs that can enhance the targeting capability of oligonucleotide drugs while crossing the blood-brain barrier to improve therapeutic efficacy.

### SUMMARY OF THE INVENTION

The objective of the present invention is to provide a precisely targeting oligonucleotide drug linked based on a complementary nucleic acid backbones composite structure, and a use thereof.

The first aspect of the present invention provides a polypeptide-oligonucleotide multimer complex, wherein the complex is a multimer formed by the complex of n monomers having complementary nucleic acid backbones, and n is a positive integer from 2 to 8;
wherein the monomer comprises:
   (a) a polypeptide monomer, wherein the polypeptide monomer comprises: (a1) a polypeptide element and (a2) a first backbone nucleic acid single strand; and the polypeptide element is covalently linked to the first backbone nucleic acid single strand;
   (b) an oligonucleotide monomer, wherein the oligonucleotide monomer comprises: (b1) an oligonucleotide pharmacodynamic element and (b2) a second backbone nucleic acid single strand; and the oligonucleotide pharmacodynamic element is covalently linked to the second backbone nucleic acid single strand; and
   (c) an optional pure backbone monomer, wherein the pure backbone monomer comprises a third backbone nucleic acid single strand, and the third backbone nucleic acid single strand is not covalently linked to a targeting element (such as an antibody) nor to a pharmacodynamic element (such as an oligonucleotide drug);
and, in the multimer, the backbone nucleic acid single strand of each monomer forms a complementary double strand with the backbone nucleic acid single strands of other monomers through base pairing, thereby forming a complementary nucleic acid backbone composite structure;
and each complex carries at least one (a1) polypeptide element and at least one (b1) oligonucleotide pharmacodynamic element.

In another preferred embodiment, the polypeptide element is selected from the group consisting of: a polypeptide element having a targeting function, a polypeptide element with an extended half-life, or a combination thereof.

In another preferred embodiment, n is a positive integer from 2 to 6; more preferably, n is a positive integer from 3 to 6.

In another preferred embodiment, n is 2, 3, 4, 5, 6, 7, or 8.

In another preferred embodiment, the polypeptide element having a targeting function is selected from the group consisting of: an antibody or an active fragment thereof, a ligand or an active fragment thereof, or a combination thereof.

In another preferred embodiment, the antibody or binding fragment thereof comprises a humanized antibody or binding fragment thereof, a chimeric antibody or binding fragment thereof, a monoclonal antibody or binding fragment thereof, a monovalent Fab', a bivalent Fab2, single-chain variable fragment (scFv), tandem scFv, bispecific antibody, trispecific antibody, mini-antibody, TriBi mini-antibody, single-domain antibody (sdAb), tandem single-domain antibody (sdAb), IgG-scFv, BiTE, DART, TandAb, scDiabody, scDiabody-Fc, scDiabody-CH3, scFv-CH3 KIH, Fab-scFv-Fc, Fab-scFv-Fc KIH, Fab-scFv, scFv-CH-CL-scFv, F(ab')2-scFv2, scFv-KIH, tetravalent HCAb, intracellular antibody, or camelid antibody or binding fragment thereof.

In another preferred embodiment, the polypeptide element having a targeting function targets a target selected from the group consisting of: TfR1, BCMA, CD73, GPC3, HER2, PMSA, 4-1BB, OX40, GLP-1, Trop2, FGL1, LFA-3, 2B4, 5T4, α-4 integrin, α-V integrin, α4β7 integrin, α4β7 integrin, α-SMA, AGR2, Apelin J receptor, APRIL, B7-H3, B7-H4, BAFF, BTLA, complement C5, C-242,CA9, CA19-9, carbonic anhydrase 9, CD2, CD3, CD6, CD9, CD11a, CD19, CD20, CD22, CD24, CD25, CD27, CD30, CD33, CD38, CD40, CD40L, CD41, CD44,CD44v6, CD47, CD51, CD52, CD56, CD64, CD69, CD70, CD71, CD74, CD80, CD81, CD86, CD95, CD107a, CD117, CD123, CD125, CD132 (IL-2Rg), CD133, CD137, CD138, CD160, CD166, CD172A, CD248, CEACAM5 (CEA), CEACAM6 (NCA-90), CLAUDIN-3, CLAUDIN-4, cMet, collagen, Cripto, CSFR, CSFR-1, CTLA-4, CTGF, CXCL10, CXCL13, CXCR1, CXCR2, CXCR4, CYR61, DL44, DLK1, DLL4, DPP-4, DSG1, EDA, EDB, EGFR, EGFRviii, Endothelin B Receptor (ETBR), ENPP3, EpCAM, EPHA2, EPHB2, ERBB3, RSV F protein, FAP, FGF-2, FGF8, FGFR1, FGFR2, FGFR3, FGFR4, FLT-3, folate receptor α (FRα), FSP-1, GAL3ST1, G-CSFR, GD2, GITR, GLUT1, GLUT4, GM-CSFR, GP1lb/IIIa receptor, Gpl30, GPIIB/IIIA, GPNMB, GRP78, HER2/neu, HER3, HER4, hGH, HLA-DR, HVEM, hyaluronidase, ICOS, IgE, IgE receptor (FceRI), IGF1R, IL-1B, IL-1R, IL-12p40, IL-12R, IL-12Rβ1, IL-13R, IL-13Ra2, IL-23R, IL-27/IL-27R (wsxl), IL-29, IL-31R, IL-31/IL-31R, IL-2R, IL-4R, IL-6R, IL-1 receptor accessory protein (IL-1RAP), insulin receptor, Jagged ligand, Jagged 1, Jagged 2, KISS1-R, KLRG1, LAG-3, LIF-R, Lewis X, LIGHT, LRP4, LRRC26, Ly6G6D, LyPD1, MCSP, Mesothelin, MRP4, MUC1, Mucin-16 (MUC16, CA-125), Na/K ATPase, Nicastrin, Notch receptor, Notch 1, Notch 2, Notch 3, Notch 4, NOV, OSM-R, OX-40, PAR2, PDGF-AA, PDGF-BB, PDGFRα, PDGFRβ, PD-1, PD-L1, PD-L2, Phosphatidylserine, P1GF, PSCA, PSMA, PSGR, RAAG12, RAGE, SLC44A4, Siglecl5, STEAP1, STEAP2, TAG-72, TAPA1, TEM-8, TfR1, TGFβ, TIGIT, TIM-3, TLR2, TLR4, TLR6, TLR7, TLR8, TLR9, TMEM31, TNFR, TNFRS12A, TRAIL-R1, TRAIL-R2, transferrin, transferrin receptor, TRK-A, TRK-B, uPAR, VAP1, VCAM-1, VEGFR1, VEGFR2, VEGFR3, VISTA, WISP-1, WISP-2, WISP-3, or combinations thereof.

In another preferred embodiment, the polypeptide element having a targeting function is selected from the group consisting of: single-domain antibody (VHH), single-chain antibody, natural ligand, or combinations thereof.

In another preferred embodiment, the polypeptide element having a targeting function comprises an antibody or a binding fragment thereof that targets TfR1.

In another preferred embodiment, the polypeptide element having a targeting function comprises a single-domain antibody that targets TfR1.

In another preferred embodiment, the polypeptide element having a targeting function has the sequence shown in SEQ ID NOs: 361.

In another preferred embodiment, the polypeptide element with an extended half-life is selected from the group consisting of: native albumin, recombinant albumin, anti-albumin antibody (including single-domain antibody, single-chain antibody, Fab, and monoclonal antibody), protein that directly binds to FcRn, any protein with a long half-life, or combinations thereof.

In another preferred embodiment, the polypeptide element with an extended half-life comprises a single-domain antibody against albumin.

In another preferred embodiment, the polypeptide element with an extended half-life has the sequence shown in SEQ ID NOs: 362.

In another preferred embodiment, the complex comprises 1, 2, or 3 different types of polypeptide monomers.

In another preferred embodiment, the different types are distinguished solely based on the polypeptide elements.

In another preferred embodiment, the complex comprises both a polypeptide element having a targeting function and a polypeptide element with an extended half-life.

In another preferred embodiment, the complex comprises both a single-domain antibody that targets TfR1 and an anti-albumin single-domain antibody.

In another preferred embodiment, the oligonucleotide pharmacodynamic element is selected from the group consisting of: antisense oligonucleotides (ASOs), short interfering nucleic acids (siNAs), short interfering RNAs (siRNAs), double-stranded RNAs (dsRNAs), microRNAs (miRNAs), short hairpin RNAs (shRNAs), PMOs, or mRNAs, or combinations thereof.

The present invention also provides a polypeptide-oligonucleotide multimer complex, wherein the complex carries at least one (a1) polypeptide element and at least one (b1) oligonucleotide pharmacodynamic element, and the (a1) polypeptide element and (b1) oligonucleotide pharmacodynamic element are conjugated together via a complementary nucleic acid backbone composite structure;
the complementary nucleic acid backbone composite structure is formed by the complex of n complementary nucleic acid backbone monomers having a complementary nucleic acid backbone pairing region, where n is a positive integer from 2 to 8;
furthermore, each of the single nucleic acid strand of complementary nucleic acid backbone monomer forms a complementary double strand with a single nucleic acid strand of the other complementary nucleic acid backbone monomers through base pairing, thereby forming the complementary nucleic acid backbone composite structure.

In another preferred embodiment, the complex comprises at least one polypeptide element having a targeting function and at least one oligonucleotide pharmacodynamic element having a pharmacodynamic function.

In another preferred embodiment, the polypeptide element is capable of specifically binding to a cell surface receptor and being internalized into the cell.

In another preferred embodiment, the polypeptide element optionally further comprises an extended half-life element.

In another preferred embodiment, the polypeptide monomer is not covalently linked to the oligonucleotide pharmacodynamic element.

In another preferred embodiment, the polypeptide monomer is covalently linked to the oligonucleotide pharmacodynamic element.

In another preferred embodiment, the polypeptide monomer has the structure of Formula I:

Z1-W1 (I)

wherein,
Z1 is a polypeptide moiety;
W1 is a first backbone nucleic acid single strand;
"-" represents a linker or bond.

In another preferred embodiment, the W1 comprises a pairing region of the complementary nucleic acid backbone.

In another preferred embodiment, the oligonucleotide monomer has the structure of Formula II:

Y1-W2-Y2 (II)

wherein,
Y1 is absent or a first oligonucleotide pharmacodynamic element;
Y2 is absent or a second oligonucleotide pharmacodynamic element;
W2 is a second backbone nucleic acid single strand;
"-" represents a linker or bond;
wherein Y1 and Y2 are not both absent, and the first oligonucleotide pharmacodynamic element and the second oligonucleotide pharmacodynamic element are the same or different.

In another preferred embodiment, W2 comprises a pairing region of complementary nucleic acid backbones.

In another preferred embodiment, the pure backbone monomer has the structure of Formula III:

V1-W3-V2 (III)

wherein,
V1 is absent or a first detectable labeling group;
V2 is absent or a second detectable labeling group;
W3 is a third backbone nucleic acid single strand;
"-" is a linker or bond.

In another preferred embodiment, V1 and V2 are both absent.

In another preferred embodiment, the first detectable labeling group and the second detectable labeling group are the same or different.

In another preferred embodiment, the backbone nucleic acid single strand is selected from the group consisting of: L-nucleic acids, peptide nucleic acids, locked nucleic acids, thio-modified nucleic acids, 2'-fluoro-modified nucleic acids, 5-hydroxymethylcytosine nucleic acids, phosphorodiamidate morpholino nucleic acids, L-phosphorodiamidate morpholino nucleic acids, or combinations thereof.

In another preferred embodiment, the complex comprises the structure shown in Formula i:

(A-Y-)m-PNSC-(-Z-R)n i

wherein,
A comprises a polypeptide element having a targeting function;
R comprises an oligonucleotide pharmacodynamic element;
PNSC is a complementary nucleic acid backbone composite structure;
Y is a bond or a first linker;
Z is a bond or a second linker; and
m and n are each independently a positive integer of ≥1.

In another preferred embodiment, n ≥ 2; preferably, n ≥ 3; more preferably, n ≥ 4.

In another preferred embodiment, when m ≥ 2, the polypeptide components in the complex may be the same or different.

In another preferred embodiment, m is 2, and the oligonucleotide complex comprises A1 and A2, where A1 is an antibody targeting TfR1 and A2 is an anti-albumin antibody.

In another preferred embodiment, when n ≥ 2, the oligonucleotide pharmacodynamic elements in the complex may be the same or different.

In another preferred embodiment, the ratio of n to m is 10:1, more preferably 4:1, and most preferably 2:1.

In another preferred embodiment, the first linker is selected from: a cleavable linker and a non-cleavable linker; preferably, the first linker is a non-cleavable linker.

In another preferred embodiment, the second linker is selected from: a cleavable linker and a non-cleavable linker; preferably, the second linker is a cleavable linker.

In another preferred embodiment, the cleavable linker is selected from the group consisting of: MC-VC-PAB, hydrazone linkers, disulfide (selenide) linkers, boronates, thioketals, azoketones, polypeptide substrates, enzyme-cleavable linkers, or combinations thereof.

In another preferred embodiment, the non-cleavable linker is selected from the group consisting of: SMCC, an amino-to-amino-coupled bifunctional non-cleavable linker, an amino-to-thiol-coupled bifunctional non-cleavable linker, or a thiol-to-thiol-coupled bifunctional non-cleavable linker.

In another preferred embodiment, the oligonucleotide pharmacodynamic element comprises at least about 10 to about 30 nucleotides in length.

In another preferred embodiment, the oligonucleotide pharmacodynamic element comprises modified nucleotides.

In another preferred embodiment, the oligonucleotide pharmacodynamic element comprises at least one 2'-modified nucleotide and at least one linker between modified nucleotides.

In another preferred embodiment, the 2'-modified nucleotides include morpholino, 2'-O-methyl, 2'-O-methoxyethyl (2'-O-MOE), 2'-O-aminopropyl, 2'-deoxy, T-deoxy-2'-fluoro, 2'-O-aminopropyl (2'-O-AP), 2'-O-dimethylaminoethyl (2'-O-DMAOE), 2'-O-dimethylaminopropyl (2'-ODMAP), TO-dimethylaminoethoxyethyl (2'-O-DMAEOE) or 2'-O-N-methylacetamido (2'-O-NMA)-modified nucleotides.

In another preferred embodiment, the 2'-modified nucleotides include locked nucleic acids (LNA), ethylene nucleic acids (ENA), or peptide nucleic acids (PNA).

In another preferred embodiment, the oligonucleotide pharmacodynamic element comprises one or more morpholino-modified nucleotides.

In another preferred embodiment, the oligonucleotide pharmacodynamic element comprises one or more phosphorodiamidate morpholino oligonucleotides.

In another preferred embodiment, the oligonucleotide pharmacodynamic element comprises at least 1, 2, 3, 4, 5, 6, 7, 8, 9, 10, 11, 12, 13, 14, 15, 16, 17, 18, 19, 20, or more phosphorodiamidate morpholino oligonucleotides.

In another preferred embodiment, the complex comprises an oligonucleotide pharmacodynamic element containing the nucleotide sequence shown in SEQ ID NOs: 363, and/or an oligonucleotide pharmacodynamic element containing the nucleotide sequence shown in SEQ ID NOs: 364.

In another preferred embodiment, the complex simultaneously comprises an oligonucleotide pharmacodynamic element containing the nucleotide sequence shown in SEQ ID NOs: 363 and an oligonucleotide pharmacodynamic element containing the nucleotide sequence shown in SEQ ID NOs: 364.

In another preferred embodiment, the nucleotide sequence comprises one or more phosphorodiamidate morpholino-modified nucleotides.

In another preferred embodiment, the nucleotide sequence comprises at least about 15%, 20%, 30%, 40%, 50%, 60%, 70%, 80%, 90%, 95%, 99%, or 100% phosphorodiamidate morpholino-modified nucleotides.

In another preferred embodiment, the PNSC comprises the structure shown in Formula 1:

C1-R1-C2-R2-C3 (1)

wherein,
R1 is complementary base pairing region 1;
R2 is complementary base pairing region 2;
C1, C2, and C3 are each, independently absent or a redundant nucleic acid;
"-" represents a bond.

In another preferred embodiment, the lengths of R1 and R2 are each, independently, 10-20 bases, more preferably 14-16 bases.

In another preferred embodiment, the length of C1 is 0-5 bases.

In another preferred embodiment, the length of C3 is 0-5 bases.

In another preferred embodiment, the length of C2 is 0-3 bases.

In another preferred embodiment, the sequence of C2 is selected from the group consisting of: A, AA, AGA, or AAA.

In another preferred embodiment, R1 of each monomer forms a complementary base pairing structure with R2 of the monomer immediately to its left (or on its left side); and R2 forms a complementary base pairing structure with R1 of the monomer immediately to its right (or on its right side).

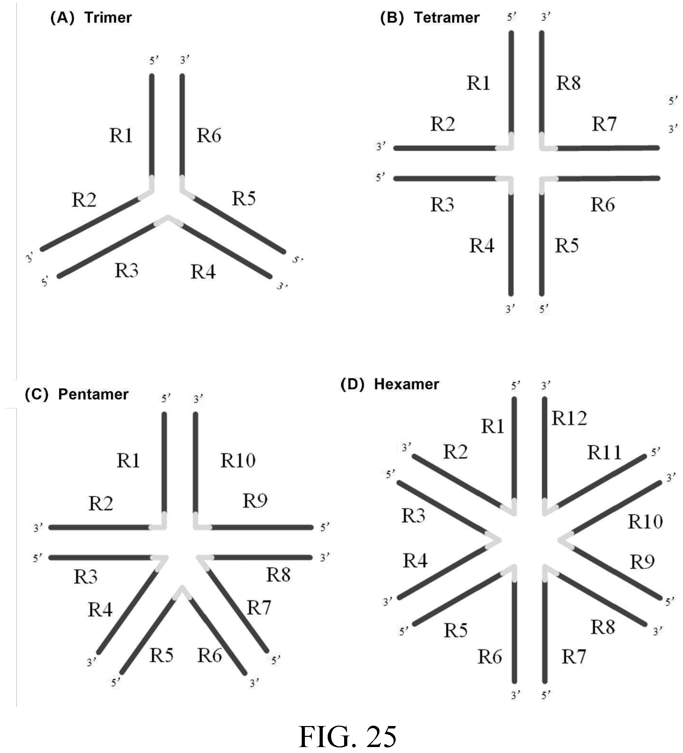

In another preferred embodiment, the PNSC is a trimer, tetramer, pentamer, or hexamer; more preferably, the structure of the PNSC is as shown in Figure 25.

In another preferred embodiment, the multimer comprises a backbone nucleic acid single strand sequence set selected from Table A or Table B:

**Table A**

| Sequence Set 3-1 Number | Optimized Sequence | SEQ ID No.: |
|---|---|---|
| *S*₁ | ACACCTGGTTGTTGGATAAATCGTTGAAGGCTAGGA | 1 |
| *S*₂ | ATCCTAGCCTTCAACGAAAAAACTAGAGTCCGCCGA | 2 |
| *S*₃ | ATCGGCGGACTCTAGTTAAAATCCAACAACCAGGTG | 3 |

| Sequence Set 3-2 Number | Optimized Sequence | |
|---|---|---|
| *S*₁ | ATGCGTTGAGTTCCAGTAAAGGCAACATCACCACAT | 4 |
| *S*₂ | AATGTGGTGATGTTGCCAAATCTGAATCCTCGTGCT | 5 |
| *S*₃ | AAGCACGAGGATTCAGAAAAACTGGAACTCAACGCA | 6 |

| Sequence Set 3-3 Number | Optimized Sequence | |
|---|---|---|
| *S*₁ | ATTCCAATCGTCCTGTGAAAAGTTCCGCTCTGAGTT | 7 |
| *S*₂ | AAACTCAGAGCGGAACTAAACTGGCAGATGGATGAA | 8 |
| *S*₃ | ATTCATCCATCTGCCAGAAACACAGGACGATTGGAA | 9 |

| Sequence Set 3-4 Number | Optimized Sequence | |
|---|---|---|
| *S*₁ | ACGAGGCAAGTTCTGTGAAAATGACTACCAGGTCCG | 10 |
| *S*₂ | ACGGACCTGGTAGTCATAAAATCCACTGACGCTGAA | 11 |
| *S*₃ | ATTCAGCGTCAGTGGATAAACACAGAACTTGCCTCG | 12 |

| Sequence Set 3-5 Number | Optimized Sequence | |
|---|---|---|
| *S*₁ | ATAGTTCGTTGCTCGGAAAAGGCATTGAGAGGACCT | 13 |
| *S*₂ | AAGGTCCTCTCAATGCCAAAATGGTGATGTCGCTTG | 14 |
| *S*₃ | ACAAGCGACATCACCATAAATCCGAGCAACGAACTA | 15 |

| Sequence Set 3-6 Numbering | Optimized Sequence | |
|---|---|---|
| *S*₁ | AGTCGTGTGCTTCCAAGAAATAGCCAGGTGAGGACT | 16 |
| *S*₂ | AAGTCCTCACCTGGCTAAAAAACAGCGGAGTGTCAT | 17 |
| *S*₃ | AATGACACTCCGCTGTTAAACTTGGAAGCACACGAC | 18 |

| Sequence Set 3-7 Number | Optimized Sequence | |
|---|---|---|
| *S*₁ | AACGCATCGCTTGATAGAAAAGAGGAGCACGGTTAT | 19 |
| *S*₂ | AATAACCGTGCTCCTCTAAAGTAGGCAATCCACCAT | 20 |
| *S*₃ | AATGGTGGATTGCCTACAAACTATCAAGCGATGCGT | 21 |

| Sequence Set 3-8 Number | Optimized Sequence | |
|---|---|---|
| *S*₁ | AGTCGTTCCACCGAACAAAATGGCTCTGGTCATTGA | 22 |
| *S*₂ | ATCAATGACCAGAGCCAAAAAATCGCACATCTCAGG | 23 |
| *S*₃ | ACCTGAGATGTGCGATTAAATGTTCGGTGGAACGAC | 24 |

| Sequence Set 3-9 Number | Optimized Sequence | |
|---|---|---|
| *S*₁ | AGCGGAGTGACCATAGTAAAAGGCAGGACATTGTTC | 25 |
| *S*₂ | AGAACAATGTCCTGCCTAAAGTGCTCGTCGTGAAGA | 26 |
| *S*₃ | ATCTTCACGACGAGCACAAAACTATGGTCACTCCGC | 27 |

| Sequence Set 3-10 Number | Optimized Sequence | |
|---|---|---|
| *S*₁ | AATTGGACCGCTCTACTAAAATGGCACCACAGTCAA | 28 |
| *S*₂ *S*₃ | ATTGACTGTGGTGCCATAAACAGGCTATCAGCATCC | 29 |
| | AGGATGCTGATAGCCTGAAAAGTAGAGCGGTCCAAT | 30 |

| Sequence Set 3-11 Number | Optimized Sequence | |
|---|---|---|
| *S*₁ | ACCATTGAGCCAGTGATAAAAACCGTTGTGAGTTGC | 31 |
| *S*₂ | AGCAACTCACAACGGTTAAATCGCACACCTGTCGTA | 32 |
| *S*₃ | ATACGACAGGTGTGCGAAAAATCACTGGCTCAATGG | 33 |

| Sequence Set 3-12 Number | Optimized Sequence | |
|---|---|---|
| *S*₁ | AAGTGAAGAAGCAGCCTAAAGTTGTCATCGCACACC | 34 |
| *S*₂ | AGGTGTGCGATGACAACAAAATGTCGTAACCGTGGA | 35 |
| *S*₃ | ATCCACGGTTACGACATAAAAGGCTGCTTCTTCACT | 36 |

| Sequence Set 3-13 Number | Optimized Sequence | |
|---|---|---|
| *S*₁ | AATAGCGTCTTGAGCCTAAATGGAGGACATACCGAC | 37 |
| *S*₂ | AGTCGGTATGTCCTCCAAAAGGTCACAGTTGCTGCT | 38 |
| *S*₃ | AAGCAGCAACTGTGACCAAAAGGCTCAAGACGCTAT | 39 |

| Sequence Set 3-14 Number | Optimized Sequence | |
|---|---|---|
| *S*₁ | ATGCCGTGTTCAGATTCAAATGTGCGTCTGGATTGA | 40 |
| *S*₂ | ATCAATCCAGACGCACAAAAAGACAGGTGGTCCGAT | 41 |
| *S*₃ | AATCGGACCACCTGTCTAAAGAATCTGAACACGGCA | 42 |

| Sequence Set 3-15 Number | Optimized Sequence | |
|---|---|---|
| *S*₁ | ATTCAGGACAGCGTCATAAAACCGACTGGAGCAACT | 43 |
| *S*₂ | AAGTTGCTCCAGTCGGTAAAGATGCCTTCGTGTGAG | 44 |
| *S*₃ | ACTCACACGAAGGCATCAAAATGACGCTGTCCTGAA | 45 |

| Sequence Set 3-16 Number | Optimized Sequence | |
|---|---|---|
| *S*₁ | AGCAGCCAAGGTTATCTAAACAATGACACGGAGGAT | 46 |
| *S*₂ *S*₃ | AATCCTCCGTGTCATTGAAAGTGATTCGCACCAGAC | 47 |
| | AGTCTGGTGCGAATCACAAAAGATAACCTTGGCTGC | 48 |

| Sequence Set 3-17 Number | Optimized Sequence | |
|---|---|---|
| *S*₁ | ACCACCGTGTATGACCTAAAAGTGACAGCACATCGC | 49 |
| *S*₂ *S*₃ | AGCGATGTGCTGTCACTAAAACAGGCTCTACGAGGA | 50 |
| | ATCCTCGTAGAGCCTGTAAAAGGTCATACACGGTGG | 51 |

| Sequence Set 3-18 Number | Optimized Sequence | |
|---|---|---|
| *S*₁ | AACTACGGAGCGAAGATAAATCCTGACCAACTTGCT | 52 |
| *S*₂ | AAGCAAGTTGGTCAGGAAAAGACTGGCTGAACACGA | 53 |
| *S*₃ | ATCGTGTTCAGCCAGTCAAAATCTTCGCTCCGTAGT | 54 |

| Sequence Set 3-19 Number | Optimized Sequence | |
|---|---|---|
| *S*₁ | AGTTCCTGATCCAGCCTAAACATCCTTGTCTTGCCA | 55 |
| *S*₂ | ATGGCAAGACAAGGATGAAACACGACCGCTTAGAAG | 56 |
| *S*₃ | ACTTCTAAGCGGTCGTGAAAAGGCTGGATCAGGAAC | 57 |

| Sequence Set 3-20 Number | Optimized Sequence | |
|---|---|---|
| *S*₁ | ATATCGCACTCCAGCATAAACCGTGTGAACATCAGG | 58 |
| *S*₂ | ACCTGATGTTCACACGGAAAAGCCTACGAGACTTGG | 59 |
| *S*₃ | ACCAAGTCTCGTAGGCTAAAATGCTGGAGTGCGATA | 60 |
| | Optimized Sequence | SEQ ID No: |
| *S*₁ | AAGCGTCGTGAATCCAAATGAGCCTGCCAATG | 61 |
| *S*₂ | ACATTGGCAGGCTCAAAACCGAAGTCAACGCT | 62 |
| *S*₃ | AAGCGTTGACTTCGGAAAACTATGGACGGCGA | 63 |
| *S*₄ | ATCGCCGTCCATAGTAAAGGATTCACGACGCT | 64 |

| | Optimized Sequence | |
|---|---|---|
| *S*₁ | AATGGCGAGCAATCCAAATGAGCCTGGACCAA | 65 |
| *S*₂ | ATTGGTCCAGGCTCAAAACCGAACGCTGTGAT | 66 |
| *S*₃ | AATCACAGCGTTCGGAAAACTATCGTGCGGCA | 67 |
| *S*₄ | ATGCCGCACGATAGTAAAGGATTGCTCGCCAT | 68 |

| | Optimized Sequence | |
|---|---|---|
| *S*₁ | ATGACCACGCAATCCAAATGAGCCAACCTCCA | 69 |
| *S*₂ | ATGGAGGTTGGCTCAAAACCGAACAGCAGCTT | 70 |
| *S*₃ | AAAGCTGCTGTTCGGAAAACTATCTGCCGCCT | 71 |
| *S*₄ | AAGGCGGCAGATAGTAAAGGATTGCGTGGTCA | 72 |

| | Optimized Sequence | |
|---|---|---|
| *S*₁ | ATGTCGCACCAATCCAAATGAGCAAGCCTCGT | 73 |
| *S*₂ | AACGAGGCTTGCTCAAAACCGAACGCTGTCAT | 74 |
| *S*₃ | AATGACAGCGTTCGGAAAACTATGTGGCGGCA | 75 |
| *S*₄ | ATGCCGCCACATAGTAAAGGATTGGTGCGACA | 76 |

| | Optimized Sequence | |
|---|---|---|
| *S*₁ | ATGCTGGCACAATCCAAATGAGCGACGAGGTT | 77 |
| *S*₂ | AAACCTCGTCGCTCAAAACCGAAGTGCCAGTT | 78 |
| *S*₃ | AAACTGGCACTTCGGAAAACTATGAGGCGGCT | 79 |
| *S*₄ | AAGCCGCCTCATAGTAAAGGATTGTGCCAGCA | 80 |

| | Optimized Sequence | |
|---|---|---|
| *S*₁ | ATGTCGCACCAATCCAAATGAGCAGGTTGGCA | 81 |
| *S*₂ | ATGCCAACCTGCTCAAAACCGAACGCTGTCAA | 82 |
| *S*₃ | ATTGACAGCGTTCGGAAAACTATCAGCCGCCT | 83 |
| *S*₄ | AAGGCGGCTGATAGTAAAGGATTGGTGCGACA | 84 |

| | Optimized Sequence | |
|---|---|---|
| *S*₁ | ATGTGGTCGCAATCCAAATGAGCACCTGCCAA | 85 |
| *S*₂ | ATTGGCAGGTGCTCAAAACCGAACGTGACGAT | 86 |
| *S*₃ | AATCGTCACGTTCGGAAAACTATCAACGCCGC | 87 |
| *S*₄ | AGCGGCGTTGATAGTAAAGGATTGCGACCACA | 88 |

| | Optimized Sequence | |
|---|---|---|
| *S*₁ | AAGCGTCGTCAATCCAAATGAGCACGGCAATG | 89 |
| *S*₂ | ACATTGCCGTGCTCAAAACCGAAGTGAACGCT | 90 |
| *S*₃ | AAGCGTTCACTTCGGAAAACTATGGCTCGCCT | 91 |
| *S*₄ | AAGGCGAGCCATAGTAAAGGATTGACGACGCT | 92 |
| | Optimized Sequence | |

| *S*₁ | ATGTGGCGACAATCCAAATGAGCAAGCCTCCA | 93 |
|---|---|---|
| *S*₂ *S*₃ | ATGGAGGCTTGCTCAAAACCGAAGACGCTGTT | 94 |
| | AAACAGCGTCTTCGGAAAACTATCGTGCGGCA | 95 |
| *S*₄ | ATGCCGCACGATAGTAAAGGATTGTCGCCACA | 96 |

| | Optimized Sequence | |
|---|---|---|
| *S*₁ | ATGCTGCCACAATCCAAATGAGCCTGGAACCA | 97 |
| *S*₂ | ATGGTTCCAGGCTCAAAACCGAACGCAGTCAT | 98 |
| *S*₃ | AATGACTGCGTTCGGAAAACTATCGCCGCTCT | 99 |
| *S*₄ | AAGAGCGGCGATAGTAAAGGATTGTGGCAGCA | 100 |

| | Optimized Sequence | |
|---|---|---|
| *S*₁ | ATGCGTCGTCAATCCAAATGAGCTTGGCAAGG | 101 |
| *S*₂ | ACCTTGCCAAGCTCAAAACCGAACGTGCTGTT | 102 |
| *S*₃ | AAACAGCACGTTCGGAAAACTATGGAGCGGCT | 103 |
| *S*₄ | AAGCCGCTCCATAGTAAAGGATTGACGACGCA | 104 |

| | Optimized Sequence | |
|---|---|---|
| *S*₁ | AACTGCCAGCAATCCAAATGAGCCTCGTTCCA | 105 |
| *S*₂ | ATGGAACGAGGCTCAAAACCGAAGTTGGCAGT | 106 |
| *S*₃ | AACTGCCAACTTCGGAAAACTATCGCCGCTTG | 107 |
| *S*₄ | ACAAGCGGCGATAGTAAAGGATTGCTGGCAGT | 108 |

| | Optimized Sequence | |
|---|---|---|
| *S*₁ | ATGCGTCGTCAATCCAAATGAGCCTCCAGGTT | 109 |
| *S*₂ | AAACCTGGAGGCTCAAAACCGAATGACACGCT | 110 |
| *S*₃ | AAGCGTGTCATTCGGAAAACTATGGCGGCAGT | 111 |
| *S*₄ | AACTGCCGCCATAGTAAAGGATTGACGACGCA | 112 |

| | Optimized Sequence | |
|---|---|---|
| *S*₁ | AAGCGTCGTGAATCCAAATGAGCCATCGTCCA | 113 |
| *S*₂ | ATGGACGATGGCTCAAAACCGAATGTGCTGGT | 114 |
| *S*₃ | AACCAGCACATTCGGAAAACTATGCGGCAACC | 115 |
| *S*₄ | AGGTTGCCGCATAGTAAAGGATTCACGACGCT | 116 |

| | Optimized Sequence | |
|---|---|---|
| *S*₁ | ATTGCCAGGATGCTGAATCACGGTCGGACA | 117 |
| *S*₂ | ATGTCCGACCGTGATAGTCGCAGAAGGCAT | 118 |
| *S*₃ | AATGCCTTCTGCGACATAGTACAACGCCGC | 119 |
| *S*₄ | AGCGGCGTTGTACTAACAGCATCCTGGCAA | 120 |

| | Optimized Sequence | |
|---|---|---|
| *S*₁ | AGGCGATCACAATCCAAATGAGCGTGTTACGG | 121 |
| *S*₂ | ACCGTAACACGCTCAAAACCGAAGTGCCAATT | 122 |
| *S*₃ | AAATTGGCACTTCGGAAAACTATGCGGCTGCT | 123 |
| *S*₄ | AAGCAGCCGCATAGTAAAGGATTGTGATCGCC | 124 |

| | Optimized Sequence | |
|---|---|---|
| *S*₁ | ATGGTCCAACACGCTAAGCCTCACCGTCTT | 125 |
| *S₂* | AAAGACGGTGAGGCTATCGCACAACCTGGT | 126 |
| *S*₃ | AACCAGGTTGTGCGAATCGGAGTGGCAGAA | 127 |
| *S*₄ | ATTCTGCCACTCCGAAAGCGTGTTGGACCA | 128 |

| | Optimized Sequence | |
|---|---|---|
| *S*₁ | AACCTTGGTGTGCGAAACTCCTGGCAGCAA | 129 |
| *S*₂ | ATTGCTGCCAGGAGTAAGCGTGTGGTTCCA | 130 |
| *S*₃ | ATGGAACCACACGCTATGAGGACCGTCGTT | 131 |
| *S*₄ | AAACGACGGTCCTCAATCGCACACCAAGGT | 132 |

| | Optimized Sequence | |
|---|---|---|
| *S*₁ | ATGCCAAGTCCGAGAATGCTGCGAACTGGT | 133 |
| *S*₂ | AACCAGTTCGCAGCAAAGAGCCTGAACCGT | 134 |
| *S*₃ | AACGGTTCAGGCTCTAACGACGCTTGACCA | 135 |
| *S*₄ | ATGGTCAAGCGTCGTATCTCGGACTTGGCA | 136 |

| | Optimized Sequence | |
|---|---|---|
| *S*₁ | AAGCAGCCTCGTTGAATCGCCAAGACACCT | 137 |
| *S*₂ | AAGGTGTCTTGGCGAAAGTTGCTCCGACGA | 138 |
| *S*₃ | ATCGTCGGAGCAACTAAGCGGTTCTGTGGA | 139 |
| *S*₄ | ATCCACAGAACCGCTATCAACGAGGCTGCT | 140 |

| | Optimized Sequence | |
|---|---|---|
| *S*₁ | ATCAGGCGACCTCTTAAAACCACCATCGTTGC | 141 |
| *S*₂ | AGCAACGATGGTGGTAAAAATCCAAATGAGCGTGTT | 142 |
| | ACGG | |
| *S*₃ | ACCGTAACACGCTCAAAACCGAAGTGCCAATT | 143 |
| *S*₄ | AAATTGGCACTTCGGAAAACTATGCGGCTGCT | 144 |
| *S*₅ | AAGCAGCCGCATAGTAAAGGATTAAAAAGAGGTCGC | 145 |
| | CTGA | |

| | Optimized Sequence | |
|---|---|---|
| *S*₁ | AGGCGACGATGTCTTAAAACCTGGTTGCTGGA | 146 |
| *S*₂ | ATCCAGCAACCAGGTAAAAATCCAAATGAGCGTGTT | 147 |
| | ACGG | |
| *S*₃ | ACCGTAACACGCTCAAAACCGAAGTGCCAATT | 148 |
| *S*₄ | AAATTGGCACTTCGGAAAACTATGCGGCTGCT | 149 |
| *S*₅ | AAGCAGCCGCATAGTAAAGGATTAAAAAGACATCGT | 150 |
| | CGCC | |

| | Optimized Sequence | |
|---|---|---|
| *S*₂ | ATGGAACCTGGTGCTAAATGCTCGCCTGTCAA | 151 |
| | ATTGACAGGCGAGCAAAAAATCCAAATGAGCGTGTT | 152 |
| | ACGG | |
| *S*₃ | ACCGTAACACGCTCAAAACCGAAGTGCCAATT | 153 |
| *S*₄ | AAATTGGCACTTCGGAAAACTATGCGGCTGCT | 154 |
| *S*₅ | AAGCAGCCGCATAGTAAAGGATTAAAAGCACCAGGT | 155 |
| | TCCA | |

| | Optimized Sequence | |
|---|---|---|
| *S*₁ | ATGGTCAGGCGACTTAAAAGGACGAGGTTGCT | 156 |
| *S*₂ | AAGCAACCTCGTCCTAAAAATCCAAATGAGCGTGTT ACGG | 157 |
| *S*₃ | ACCGTAACACGCTCAAAACCGAAGTGCCAATT | 158 |
| *S*₄ | AAATTGGCACTTCGGAAAACTATGCGGCTGCT | 159 |
| *S*₅ | AAGCAGCCGCATAGTAAAGGATTAAAAAGTCGCCTG | 160 |
| | ACCA | |
| *S*₁ | ATGCTGGACCACCTTAAATCAGATGGAGGCGA | 161 |
| *S*₂ | ATCGCCTCCATCTGAAAAAATCCAAATGAGCGTGTTA | 162 |
| | CGG | |
| *S*₃ | ACCGTAACACGCTCAAAACCGAAGTGCCAATT | 163 |
| *S*₄ | AAATTGGCACTTCGGAAAACTATGCGGCTGCT | 164 |
| *S*₅ | AAGCAGCCGCATAGTAAAGGATTAAAAAGGTGGTCC | 165 |
| | AGCA | |

| | Optimized Sequence | |
|---|---|---|
| *S*₁ | AAACGTCCAGGAGCTAAATCTCGTCGCCTGAA | 166 |
| *S*₂ | ATTCAGGCGACGAGAAAAAATCCAAATGAGCGTGTT | 167 |
| | ACGG | |
| *S*₃ | ACCGTAACACGCTCAAAACCGAAGTGCCAATT | 168 |
| *S*₄ | AAATTGGCACTTCGGAAAACTATGCGGCTGCT | 169 |
| *S*₅ | AAGCAGCCGCATAGTAAAGGATTAAAAGCTCCTGGA | 170 |
| | CGTT | |

| | Optimized Sequence | |
|---|---|---|
| *S*₁ | ACCACGACCATTGCTAAAAACTTCAGGCGACG | 171 |
| *S*₂ | ACGTCGCCTGAAGTTAAAAATCCAAATGAGCGTGTT ACGG | 172 |
| *S*₃ | ACCGTAACACGCTCAAAACCGAAGTGCCAATT | 173 |
| *S*₄ | AAATTGGCACTTCGGAAAACTATGCGGCTGCT | 174 |
| *S*₅ | AAGCAGCCGCATAGTAAAGGATTAAAAGCAATGGTC | 175 |
| | GTGG | |

| | Optimized Sequence | |
|---|---|---|
| *S*₁ | AAGGCGAGGTCTTCAAAATGGTTGCTGGACGA | 176 |
| *S*₂ | ATCGTCCAGCAACCAAAAAATCCAAATGAGCGTGTT | 177 |
| | ACGG | |
| *S*₃ | ACCGTAACACGCTCAAAACCGAAGTGCCAATT | 178 |
| *S*₄ | AAATTGGCACTTCGGAAAACTATGCGGCTGCT | 179 |
| *S*₅ | AAGCAGCCGCATAGTAAAGGATTAAATGAAGACCTC | 180 |
| | GCCT | |

| | Optimized Sequence | |
|---|---|---|
| *S*₁ | ATCAAGGCGACCAGTAAAAAGCTCCTCGACGA | 181 |
| *S*₂ | ATCGTCGAGGAGCTTAAAAATCCAAATGAGCGTGTT | 182 |
| | ACGG | |
| *S*₃ | ACCGTAACACGCTCAAAACCGAAGTGCCAATT | 183 |
| *S*₄ | AAATTGGCACTTCGGAAAACTATGCGGCTGCT | 184 |
| *S*₅ | AAGCAGCCGCATAGTAAAGGATTAAAACTGGTCGCC | 185 |
| | TTGA | |

| | Optimized Sequence | |
|---|---|---|
| *S*₁ | ATTCAGGCGACTCCTAAAAGCACGACGATGGT | 186 |
| *S*₁ | AACCATCGTCGTGCTAAAAATCCAAATGAGCGTGTT ACGG | 187 |
| *S*₃ | ACCGTAACACGCTCAAAACCGAAGTGCCAATT | 188 |
| *S*₄ | AAATTGGCACTTCGGAAAACTATGCGGCTGCT | 189 |
| *S*₅ | AAGCAGCCGCATAGTAAAGGATTAAAAGGAGTCGCC | 190 |
| | TGAA | |

| | Optimized Sequence | |
|---|---|---|
| *S*₁ | AAGCACCTGCAATCCAAATCGCCAGGACAAGT | 191 |
| *S*₂ | AACTTGTCCTGGCGAAAATGAGCAACCATGCC | 192 |
| *S*₃ | AGGCATGGTTGCTCAAAACCGAACGTCGTGAT | 193 |
| *S*₄ | AATCACGACGTTCGGAAAACTATGGAGCGGCT | 194 |
| *S*₅ | AAGCCGCTCCATAGTAAAGGATTGCAGGTGCT | 195 |

| | Optimized Sequence | |
|---|---|---|
| *S*₁ | AACCTGCTGCAATCCAAATCGCCACCTCAAGA | 196 |
| *S*₂ | ATCTTGAGGTGGCGAAAATGAGCCTGGACGTT | 197 |
| *S*₃ | AAACGTCCAGGCTCAAAACCGAACTGGTGCTT | 198 |
| *S*₄ | AAAGCACCAGTTCGGAAAACTATGCCGCTCCT | 199 |
| *S*₅ | AAGGAGCGGCATAGTAAAGGATTGCAGCAGGT | 200 |

| | Optimized Sequence | |
|---|---|---|
| *S*₁ | AAGCTGGTGCAATCCAAATCGCCTCCTGACAA | 201 |
| *S*₂ | ATTGTCAGGAGGCGAAAATGAGCAAGGTTGGC | 202 |
| *S*₃ | AGCCAACCTTGCTCAAAACCGAACGCAGATGT | 203 |
| *S*₄ | AACATCTGCGTTCGGAAAACTATGGAGCGGCA | 204 |
| *S*₅ | ATGCCGCTCCATAGTAAAGGATTGCACCAGCT | 205 |

| | Optimized Sequence | |
|---|---|---|
| *S*₁ | ATGCACGCACAATCCAAATCGCCATCAGAGGT | 206 |
| *S*₂ | AACCTCTGATGGCGAAAATGAGCTGCCTCCAT | 207 |
| *S*₃ | AATGGAGGCAGCTCAAAACCGAACGTCGTCAT | 208 |
| *S*₄ | AATGACGACGTTCGGAAAACTATCGAGCGGCT | 209 |
| S₅ | AAGCCGCTCGATAGTAAAGGATTGTGCGTGCA | 210 |

| | Optimized Sequence | |
|---|---|---|
| *S*₁ | AAGCGTCGTGAATCCAAATCGCCATCAGACCA | 211 |
| *S*₂ | ATGGTCTGATGGCGAAAATGAGCAAGGCTCGT | 212 |
| *S*₃ | AACGAGCCTTGCTCAAAACCGAACCAGCTTGT | 213 |
| *S*₄ | AACAAGCTGGTTCGGAAAACTATGCGGCAGGT | 214 |
| *S*₅ | AACCTGCCGCATAGTAAAGGATTCACGACGCT | 215 |

| | Optimized Sequence | |
|---|---|---|
| *S*₁ | ATCAGCACGCAATCCAAATCGCCAGTTCAACC | 216 |
| *S*₂ | AGGTTGAACTGGCGAAAATGAGCAAGCAGGCT | 217 |
| *S*₃ | AAGCCTGCTTGCTCAAAACCGAACGTGGTGTT | 218 |
| *S*₄ | AAACACCACGTTCGGAAAACTATGGAGCGGCA | 219 |
| *S*₅ | ATGCCGCTCCATAGTAAAGGATTGCGTGCTGA | 220 |

| | Optimized Sequence | |
|---|---|---|
| *S*₁ | AAGCTGCACCAATCCAAATCGCCAGAAGGTCA | 221 |
| *S*₂ | ATGACCTTCTGGCGAAAATGAGCACGACGCAT | 222 |
| *S*₃ | AATGCGTCGTGCTCAAAACCGAACAACCTGCT | 223 |
| *S*₄ | AAGCAGGTTGTTCGGAAAACTATGGAGCGGCA | 224 |
| *S*₅ | ATGCCGCTCCATAGTAAAGGATTGGTGCAGCT | 225 |

| | Optimized Sequence | |
|---|---|---|
| *S*₁ | AACGCTCGTCAATCCAAATCGCCTCAGGACAA | 226 |
| *S*₂ | ATTGTCCTGAGGCGAAAATGAGCCAACGACCT | 227 |
| *S*₃ | AAGGTCGTTGGCTCAAAACCGAAGCTGGTGTT | 228 |
| *S*₄ | AAACACCAGCTTCGGAAAACTATGCCGCACCT | 229 |
| *S*₅ | AAGGTGCGGCATAGTAAAGGATTGACGAGCGT | 230 |

| | Optimized Sequence | |
|---|---|---|
| *S*₁ | AAGTGCGTCGAATCCAAATCGCCAAGACCTCA | 231 |
| *S*₂ | ATGAGGTCTTGGCGAAAATGAGCAGGCTGGAA | 232 |
| *S*₃ | ATTCCAGCCTGCTCAAAACCGAAGCAACGTGT | 233 |
| *S*₄ | AACACGTTGCTTCGGAAAACTATGCCGCTCCT | 234 |
| *S*₅ | AAGGAGCGGCATAGTAAAGGATTCGACGCACT | 235 |

| | Optimized Sequence | |
|---|---|---|
| *S*₁ | ATCACGCAGCAATCCAAATCGCCATCACAACG | 236 |
| *S*₂ | ACGTTGTGATGGCGAAAATGAGCACGAGCCTT | 237 |
| *S*₃ | AAAGGCTCGTGCTCAAAACCGAAGGTTGCACT | 238 |
| *S*₄ | AAGTGCAACCTTCGGAAAACTATGCCGCTCCA | 239 |
| S₅ | ATGGAGCGGCATAGTAAAGGATTGCTGCGTGA | 240 |

**Table B**

| Sequence Set 6-1 Number | Optimized Sequence | SEQ ID No.: |
|---|---|---|
| *S*₁ | ATATAGTACACCGTGAAAGTTGATCTCAGTCA | 241 |
| *S*₂ | ATGACTGAGATCAACAAACTTAGCACTAACTG | 242 |
| *S*₃ | ACAGTTAGTGCTAAGAAAGACATATACTCGCT | 243 |
| *S*₄ | AAGCGAGTATATGTCAAACCTCTTCCAGAATA | 244 |
| *S*₅ | ATATTCTGGAAGAGGAAAGCTGTCTGTAATCC | 245 |
| *S*₆ | AGGATTACAGACAGCAAACACGGTGTACTATA | 246 |

| Sequence Set 6-2 Number | Optimized Sequence | |
|---|---|---|
| *S*₁ | ATAGTTCTTCCACAGAAAGACATAATCACTCG | 247 |
| *S*₂ | ACGAGTGATTATGTCAAATGAGCAGTAGATAC | 248 |
| *S*₃ | AGTATCTACTGCTCAAAAGTTACTCCGCTTAA | 249 |
| *S*₄ | ATTAAGCGGAGTAACAAACAAGGTAGTCATCT | 250 |
| *S*₅ | AAGATGACTACCTTGAAAAGAGTTGCCTATTC | 251 |
| *S*₆ | AGAATAGGCAACTCTAAACTGTGGAAGAACTA | 252 |

| Sequence | Optimized Sequence | |
|---|---|---|
| Set 6-3 Number | | |
| *S*₁ | ATAGTTCTTCCACAGAAAGACATAATCACTCG | 253 |
| *S*₂ | ACGAGTGATTATGTCAAATGAGCAGTAGATAC | 254 |
| *S*₃ | AGTATCTACTGCTCAAAAGTTACTCCGCTTAA | 255 |
| *S*₄ | ATTAAGCGGAGTAACAAACAAGGTAGTCATCT | 256 |
| *S*₅ | AAGATGACTACCTTGAAAAGAGTTGCCTATTC | 257 |
| *S*₆ | AGAATAGGCAACTCTAAACTGTGGAAGAACTA | 258 |

| Sequence Set 6-4 Number | Optimized Sequence | |
|---|---|---|
| *S*₁ | ACTTAACTTGTAGCGAAAGAATACCTGCTTCT | 259 |
| *S*₂ | AAGAAGCAGGTATTCAAATGAGGCAATTAACC | 260 |
| *S*₃ | AGGTTAATTGCCTCAAAAGAGCCAGTCTTATA | 261 |
| *S*₄ | ATATAAGACTGGCTCAAACATCACCTAGTAGT | 262 |
| *S*₅ | AACTACTAGGTGATGAAAAGTGACGACATTAC | 263 |
| *S*₆ | AGTAATGTCGTCACTAAACGCTACAAGTTAAG | 264 |

| Sequence Set 6-5 Number | Optimized Sequence | |
|---|---|---|
| *S*₁ | ACTCGGTCATCTTACAAATACGCAGGTATCTC | 265 |
| *S*₂ | AGAGATACCTGCGTAAAATATGGTTGCCTCAG | 266 |
| *S*₃ | ACTGAGGCAACCATAAAACAATCACTCTGTTG | 267 |
| *S*₄ | ACAACAGAGTGATTGAAAAGCATTAGGAAGTC | 268 |
| *S*₅ | AGACTTCCTAATGCTAAAACTATGTGTGACAG | 269 |
| *S*₆ | ACTGTCACACATAGTAAAGTAAGATGACCGAG | 270 |

| Sequence Set 6-6 Number | Optimized Sequence | |
|---|---|---|
| *S*₁ | AAGACAGTGTATCACAAAGCCGTATTGAGATA | 271 |
| *S*₂ | ATATCTCAATACGGCAAAGTACTAGATTCGCA | 272 |
| *S*₃ | ATGCGAATCTAGTACAAACCTTACTTCCAAGA | 273 |
| *S*₄ | ATCTTGGAAGTAAGGAAACTCGTCACAGGATA | 274 |
| *S*₅ | ATATCCTGTGACGAGAAACAGTTCTGACCTAG | 275 |
| *S*₆ | ACTAGGTCAGAACTGAAAGTGATACACTGTCT | 276 |

| Sequence | Optimized Sequence | |
|---|---|---|
| Set 6-7 Number | | |
| *S*₁ | ACTCGGTCATCTTACAAATACGCAGGTATCTC | 277 |
| *S*₂ | AGAGATACCTGCGTAAAATATGGTTGCCTCAG | 278 |
| *S*₃ | ACTGAGGCAACCATAAAACAATCACTCTGTTG | 279 |
| *S*₄ | ACAACAGAGTGATTGAAAAGCATTAGGAAGTC | 280 |
| *S*₅ | AGACTTCCTAATGCTAAAACTATGTGTGACAG | 281 |
| *S*₆ | ACTGTCACACATAGTAAAGTAAGATGACCGAG | 282 |

| Sequence Set 6-8 Number | Optimized Sequence | |
|---|---|---|
| *S*₁ | ACTTAACTTGTAGCGAAAGAATACCTGCTTCT | 283 |
| *S*₂ | AAGAAGCAGGTATTCAAATGAGGCAATTAACC | 284 |
| *S*₃ | AGGTTAATTGCCTCAAAAGAGCCAGTCTTATA | 285 |
| *S*₄ | ATATAAGACTGGCTCAAACATCACCTAGTAGT | 286 |
| *S*₅ | AACTACTAGGTGATGAAAAGTGACGACATTAC | 287 |
| *S*₆ | AGTAATGTCGTCACTAAACGCTACAAGTTAAG | 288 |

| Sequence Set 6-9 Number | Optimized Sequence | |
|---|---|---|
| *S*₁ | AGCCTGACAATACTAAAATGCTTACCTACAAG | 289 |
| *S*₂ | ACTTGTAGGTAAGCAAAAGCGTATAGAGATGA | 290 |
| *S*₃ | ATCATCTCTATACGCAAAACAGTCCACTAATG | 291 |
| *S*₄ | ACATTAGTGGACTGTAAAATTCTGTTGACTCG | 292 |
| *S*₅ | ACGAGTCAACAGAATAAACTTCAGTATCCGTC | 293 |
| *S*₆ | AGACGGATACTGAAGAAATAGTATTGTCAGGC | 294 |

| Sequence Set 6-10 Number | Optimized Sequence | |
|---|---|---|
| *S*₁ | AGGAAGACTGAGTATAAAGCTCTAATCTACGC | 295 |
| *S*₂ | AGCGTAGATTAGAGCAAACCGACACTAAGTTA | 296 |
| *S*₃ | ATAACTTAGTGTCGGAAATGATAACAGGTTGG | 297 |
| *S*₄ | ACCAACCTGTTATCAAAACTACTACCTTGTGT | 298 |
| *S*₅ | AACACAAGGTAGTAGAAAGAGACGATCAATCA | 299 |
| *S*₆ | ATGATTGATCGTCTCAAAATACTCAGTCTTCC | 300 |

| Sequence | Optimized Sequence | |
|---|---|---|
| Set 6-11 Number | | |
| *S*₁ | AATAATGCTACCTGGAAAGTTCAGCCATATAC | 301 |
| *S*₂ | AGTATATGGCTGAACAAATGTTGTCTCACACA | 302 |
| *S*₃ | ATGTGTGAGACAACAAAAGTAAGAGGACTTCT | 303 |
| S₄ | AAGAAGTCCTCTTACAAACGACATGACCACTA | 304 |
| *S*₅ | ATAGTGGTCATGTCGAAAATCTAGTCTACGCT | 305 |
| *S*₆ | AAGCGTAGACTAGATAAACCAGGTAGCATTAT | 306 |

| Sequence Set 6-12 Number | Optimized Sequence | |
|---|---|---|
| *S*₁ | ATCACGCAGCAATCCAAATCGCCATCACAACG | 307 |
| *S*₂ | ACGTTGTGATGGCGAAAATGAGCACGAGCCTT | 308 |
| *S*₃ | AAAGGCTCGTGCTCAAAACCGAAGGTTGCACT | 309 |
| *S*₄ | AAGTGCAACCTTCGGAAAACTATGCCGCTCCA | 310 |
| S₅ | ATGACAGAGTCAATGAAATCGTACTCCTATGA | 311 |
| S₆ | ATCATAGGAGTACGAAAATGGCACTTGATGTA | 312 |

| Sequence Set 6-13 Number | Optimized Sequence | |
|---|---|---|
| *S*₁ | AACGGAGTTATAGTGAAAGGATGTTAGAGACT | 313 |
| *S*₂ | AAGTCTCTAACATCCAAAATCAGAGTGCGATA | 314 |
| S₃ | ATATCGCACTCTGATAAAGGCTTATGACTGAA | 315 |
| S₄ | ATTCAGTCATAAGCCAAACCTTGTCGTACTAT | 316 |
| *S*₅ | AATAGTACGACAAGGAAATGACCAGCAGTATG | 317 |
| *S*₆ | ACATACTGCTGGTCAAAACACTATAACTCCGT | 318 |

| Sequence Set 6-14 Number | Optimized Sequence | |
|---|---|---|
| *S*₁ | ACTGCTACAATAAGGAAATACGAGCCTAAGAC | 319 |
| *S*₂ | AGTCTTAGGCTCGTAAAACCGTAGTTATGACT | 320 |
| *S*₃ | AAGTCATAACTACGGAAAGACCAGAACTTGAA | 321 |
| *S*₄ | ATTCAAGTTCTGGTCAAAACGCTTAGTCTACC | 322 |
| *S*₅ | AGGTAGACTAAGCGTAAAGTGTTCAGTAAGAG | 323 |
| S₆ | ACTCTTACTGAACACAAACCTTATTGTAGCAG | 324 |

| Sequence | Optimized Sequence | |
|---|---|---|
| Set 6-15 Number | | |
| *S*₁ | ACATAAGTCAGACCAAAACATTGAGATTACCG | 325 |
| *S*₂ | ACGGTAATCTCAATGAAAACAAGTATGAGGCA | 326 |
| *S*₃ | ATGCCTCATACTTGTAAAACTGTACGAAGCGA | 327 |
| *S*₄ | ATCGCTTCGTACAGTAAAGAATATCTGCTGGA | 328 |
| *S*₅ | ATCCAGCAGATATTCAAATAGGTGTTCAGTCT | 329 |
| *S*₆ | AAGACTGAACACCTAAAATGGTCTGACTTATG | 330 |

| Sequence Set 6-16 Number | Optimized Sequence | |
|---|---|---|
| *S*₁ | ACTAATCAGTCAGCGAAAACACCTATTCTCTG | 331 |
| *S*₂ | ACAGAGAATAGGTGTAAAACTATGACAGGATG | 332 |
| *S*₃ | ACATCCTGTCATAGTAAAGTGATAAGTGCCAT | 333 |
| S₄ | ATAGACCGTCTTAGAAAATAGCAATACTCCAG | 334 |
| *S*₅ | ACTGGAGTATTGCTAAAACGCTGACTGATTAG | 335 |
| *S*₆ | ACTAATCAGTCAGCGAAACGCTGACTGATTAG | 336 |

| Sequence Set 6-17 Number | Optimized Sequence | |
|---|---|---|
| *S*₁ | AAGCGTGACTATACTAAAAGGTACTCATCTCG | 337 |
| *S*₂ | ACGAGATGAGTACCTAAACAACCGTAGCATAA | 338 |
| *S*₃ | ATTATGCTACGGTTGAAATAATGTTCCAGCCT | 339 |
| S₄ | AAGGCTGGAACATTAAAATCTTCTGAATCCTG | 340 |
| *S*₅ | ACAGGATTCAGAAGAAAAGTGTATCACCAAGA | 341 |
| *S*₆ | ATCTTGGTGATACACAAAAGTATAGTCACGCT | 342 |

| Sequence Set 6-18 Number | Optimized Sequence | |
|---|---|---|
| *S*₁ | AACTTAGAACTCACCAAATAGAGAGTCGGTAT | 343 |
| *S*₂ | AATACCGACTCTCTAAAAGGATAGCCTCTTAA | 344 |
| *S*₃ | ATTAAGAGGCTATCCAAACAGGTTGTGTGAAT | 345 |
| *S*₄ | AATTCACACAACCTGAAAACATTAGTCTGCGT | 346 |
| *S*₅ | AACGCAGACTAATGTAAACTTGCCATCTGTAT | 347 |
| *S*₆ | AATACAGATGGCAAGAAAGGTGAGTTCTAAGT | 348 |

| Sequence | Optimized Sequence | |
|---|---|---|
| Set 6-19 Number | | |
| *S*₁ | ATAGTACAGCATACCAAACAGAAGTTAGTTCC | 349 |
| *S*₂ | AGGAACTAACTTCTGAAAGCGTATCATCACAT | 350 |
| *S*₃ | AATGTGATGATACGCAAACACTCCAAGATTCA | 351 |
| S₄ | ATGAATCTTGGAGTGAAAGTGAGTCTCTACAT | 352 |
| *S*₅ | AATGTAGAGACTCACAAACTATGACACGATTG | 353 |
| *S*₆ | ACAATCGTGTCATAGAAAGGTATGCTGTACTA | 354 |

| Sequence Set 6-20 Number | Optimized Sequence | |
|---|---|---|
| *S*₁ | ATTCGGTTCATACAGAAATAGTCAGTCCTTGA | 355 |
| *S*₂ | ATCAAGGACTGACTAAAACCACTTACAATCGT | 356 |
| *S*₃ | AACGATTGTAAGTGGAAAGAGTTAAGCCTCTC | 357 |
| S₄ | AGAGAGGCTTAACTCAAAACACTATAAGAGCG | 358 |
| *S*₅ | ACGCTCTTATAGTGTAAAGTCTACATCTGCTG | 359 |
| *S*₆ | ACAGCAGATGTAGACAAACTGTATGAACCGAA | 360 |

The second invention of the present invention provides a monomer library, wherein the monomer library comprises:
(a) a first monomer, wherein the first monomer is a polypeptide monomer comprising: (a1) a polypeptide element and (a2) a first backbone nucleic acid single strand; and wherein the polypeptide element is covalently linked to the first backbone nucleic acid single strand;
(b) a second monomer, wherein the second monomer is an oligonucleotide monomer comprising: (b1) an oligonucleotide pharmacodynamic element and (b2) a second backbone nucleic acid single strand; and wherein the oligonucleotide pharmacodynamic element is covalently linked to the second backbone nucleic acid single strand; and
(c) a third monomer, wherein the third monomer is a pure backbone monomer comprising a third backbone nucleic acid single strand, and wherein the third backbone nucleic acid single strand is not covalently linked to a targeting element (such as an antibody) nor to a pharmacodynamic element (such as an oligonucleotide drug).

In another preferred embodiment, the polypeptide element is selected from the group consisting of: a polypeptide element having a targeting function, a polypeptide element with an extended half-life, or a combination thereof.

In another preferred embodiment, the polypeptide element having a targeting function is selected from the group consisting of: an antibody or an active fragment thereof, a ligand or an active fragment thereof, or a combination thereof.

In another preferred embodiment, the antibody or binding fragment thereof comprises a humanized antibody or binding fragment thereof, a chimeric antibody or binding fragment thereof, a monoclonal antibody or binding fragment thereof, a monovalent Fab', a bivalent Fab2, single-chain variable fragments (scFv), tandem scFv, tandem scFv, bispecific antibody, trispecific antibodies, minibody, mini-antibody, TriBi mini-antibody, tandem single-domain antibody, single-domain antibody (sdAb), IgG-scFv, BiTE, DART, TandAb, scDiabody, scDiabody-Fc, scDiabody-CH3, scFv-CH3 KIH, Fab-scFv-Fc, Fab-scFv-Fc KIH, Fab-scFv, scFv-CH-CL-scFv, F(ab')2-scFv2, scFv-KIH, tetravalent HCAb, intracellular antibody, bispecific antibody, or camelid antibody or binding fragment thereof.

In another preferred embodiment, the polypeptide element having a targeting function targets a target selected from the group consisting of: TfR1, BCMA, CD73, GPC3, HER2, PMSA, 4-1BB, OX40, GLP-1, Trop2, FGL1, LFA-3, 2B4, 5T4, α-4 integrin, α-V integrin, α4β7 integrin, α4β7 integrin, α-SMA, AGR2, Apelin J receptor, APRIL, B7-H3, B7-H4, BAFF, BTLA, complement C5, C-242,CA9, CA19-9, carbonic anhydrase 9, CD2, CD3, CD6, CD9, CD11a, CD19, CD20, CD22, CD24, CD25, CD27, CD30, CD33, CD38, CD40, CD40L, CD41, CD44, CD44v6, CD47, CD51, CD52, CD56, CD64, CD69, CD70, CD71, CD74, CD80, CD81, CD86, CD95, CD107a, CD117, CD123, CD125, CD132 (IL-2Rg), CD133, CD137, CD138, CD160, CD166,CD172A, CD248, CEACAM5 (CEA), CEACAM6 (NCA-90), CLAUDIN-3, CLAUDIN-4, cMet, collagen, Cripto, CSFR, CSFR-1, CTLA-4, CTGF, CXCL10, CXCL13, CXCR1,CXCR2, CXCR4, CYR61, DL44, DLK1, DLL4, DPP-4, DSG1, EDA, EDB, EGFR, EGFRviii, Endothelin B Receptor (ETBR), ENPP3, EpCAM, EPHA2, EPHB2, ERBB3, RSV F protein, FAP, FGF-2, FGF8, FGFR1, FGFR2, FGFR3, FGFR4, FLT-3, folate receptor α (FRα), FSP-1, GAL3ST1, G-CSFR, GD2, GITR, GLUT1, GLUT4, GM-CSFR, GP1lb/IIIa receptor, Gpl30, GPIIB/IIIA, GPNMB, GRP78, HER2/neu, HER3, HER4, hGH, HLA-DR, HVEM, hyaluronidase, ICOS, IgE, IgE receptor (FceRI), IGF1R, IL-1B, IL-1R, IL-12p40,IL-12R, IL-12Rβ1, IL-13R, IL-13Ra2, IL-23R, IL-27/IL-27R (wsxl), IL-29,IL-31R, IL-31/IL-31R, IL-2R, IL-4R, IL-6R, IL-1 receptor-accessory protein (IL-1RAP), insulin receptor, Jagged ligand, Jagged 1, Jagged 2, KISS1-R, KLRG1, LAG-3,LIF-R, Lewis X, LIGHT, LRP4, LRRC26, Ly6G6D, LyPD1, MCSP, Mesothelin, MRP4, MUC1, Mucin-16 (MUC16, CA-125), Na/K ATPase, Nicastrin, Notch receptor, Notch 1, Notch 2, Notch 3, Notch 4, NOV, OSM-R, OX-40, PAR2, PDGF-AA, PDGF-BB, PDGFRα, PDGFRβ, PD-1, PD-L1, PD-L2, Phosphatidylserine, P1GF, PSCA, PSMA, PSGR, RAAG12, RAGE, SLC44A4, Siglecl5, STEAP1, STEAP2, TAG-72, TAPA1, TEM-8, TfR1, TGFβ, TIGIT, TIM-3, TLR2, TLR4, TLR6, TLR7, TLR8, TLR9, TMEM31, TNFR, TNFRS12A, TRAIL-R1, TRAIL-R2, transferrin, transferrin receptor, TRK-A, TRK-B, uPAR, VAP1,VCAM-1, VEGFR1, VEGFR2, VEGFR3, VISTA, WISP-1, WISP-2, WISP-3, or combinations thereof.

In another preferred embodiment, the polypeptide element having a targeting function is selected from the group consisting of: single-domain antibody (VHH), single-chain antibody, or a combination thereof.

In another preferred embodiment, the polypeptide element having a targeting function comprises an antibody or a binding fragment thereof that targets TfR1.

In another preferred embodiment, the polypeptide element having a targeting function comprises a single-domain antibody that targets TfR1.

In another preferred embodiment, the polypeptide element with an extended half-life is selected from the group consisting of: native albumin, recombinant albumin, anti-albumin antibody (including single-domain antibody, single-chain antibody, Fab, and monoclonal antibody), protein that directly binds to FcRn, any protein with an extended half-life, or combinations thereof.

In another preferred embodiment, the oligonucleotide pharmacodynamic element is selected from the group consisting of: antisense oligonucleotides (ASOs), short interfering nucleic acids (siNAs), short interfering RNAs (siRNAs), double-stranded RNAs (dsRNAs), microRNAs (miRNAs), short hairpin RNAs (shRNAs), PMOs, or mRNAs, or combinations thereof.

In another preferred embodiment, the backbone nucleic acid single strand is selected from the group consisting of: L-nucleic acid, peptide nucleic acid, locked nucleic acid, thio-modified nucleic acid, 2'-fluoro-modified nucleic acid, 5-hydroxymethylcytosine nucleic acid, morpholino phosphate nucleic acid, L-morpholino phosphate nucleic acid, or combinations thereof.

The third aspect of the present invention provides a pharmaceutical composition, wherein the pharmaceutical composition comprises:
(i) the polypeptide-oligonucleotide multimer complex of the first aspect of the present invention as an active ingredient; and
(ii) a pharmaceutically acceptable carrier.

In another preferred embodiment, the dosage form of the pharmaceutical composition is selected from the group consisting of: injections and lyophilized formulations.

The fourth aspect of the present invention provides a use of the polypeptide-oligonucleotide multimer complex of the first aspect of the present invention in the preparation of a medicament for treating a disease caused by abnormal gene expression.

In another preferred embodiment, the polypeptide-oligonucleotide multimer complex is used to inhibit the expression of an abnormal gene.

In another preferred embodiment, the disease caused by abnormal gene expression is selected from the group consisting of: cancer, cardiovascular disease, inflammatory disease, neuromuscular disease, hereditary disease, metabolic disease, and preventable infectious disease.

In another preferred embodiment, the disease caused by abnormal gene expression is Duchenne muscular dystrophy.

The fifth aspect of the present invention provides a method for treating diseases caused by abnormal gene expression, comprising the step of administering a therapeutically effective amount of the polypeptide-oligonucleotide multimer complex of the first aspect of the present invention or the pharmaceutical composition of the second aspect of the present invention to a subject in need.

In another preferred embodiment, the subject is a mammal, such as a human.

The sixth aspect of the present invention provides a single-stranded nucleic acid sequence set for forming a hexameric complementary nucleic acid backbone composite structure, the single-stranded nucleic acid sequence set is selected from the backbone nucleic acid single strand sequence sets listed in Table B under numbers 6-1 to 6-20.

It should be understood that, within the scope of the present invention, the aforementioned technical features of the present invention and the technical features specifically described below (e.g., in the examples) may be combined with one another to form new or preferred technical solutions. Due to space limitations, they will not be listed one by one here.

### DESCRIPTION OF THE DRAWINGS

Figure 1 shows a schematic diagram of an MAOC drug molecule (modular AOC, modular antibody oligonucleotide conjugate).

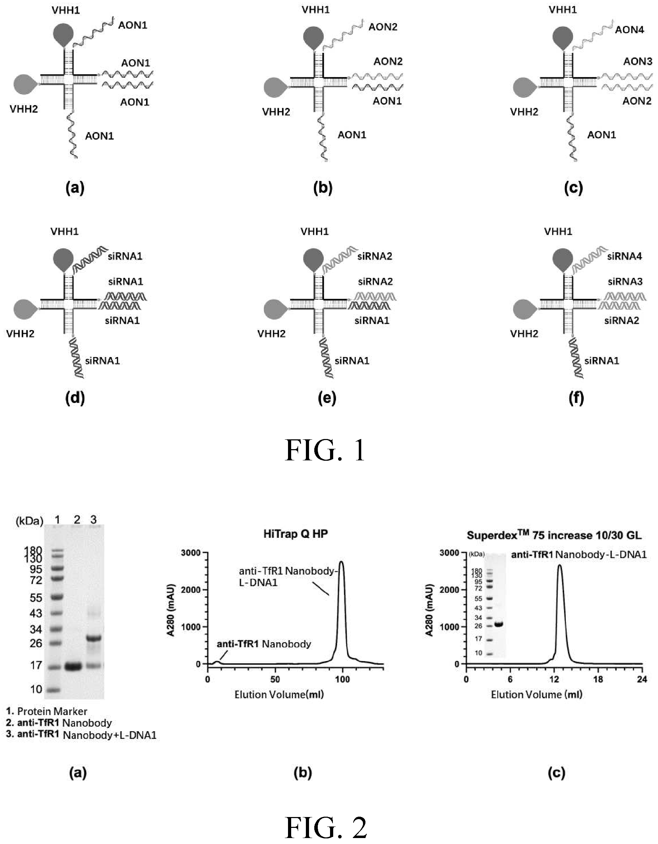

Figure 2 shows the conjugation and purification of the targeting module. (a) SDS-PAGE verification of the conjugation efficiency between the anti-TfR1 single-domain antibody (anti-TfR1 nanobody) and L-DNA1. Lane 1: Protein Marker; Lane 2: Anti-TfR1 single-domain antibody; Lane 3: Mixture of anti-TfR1 single-domain antibody and L-DNA1 after a 2-hour reaction at room temperature. (b) Free anti-TfR1 single-domain antibodies was removed from the conjugate by anion-exchange chromatography. (c) Anti-TfR1 single-domain antibody-L-DNA1 with a purity greater than 95% was simultaneously obtained using molecular sieve displacement of the sample buffer.

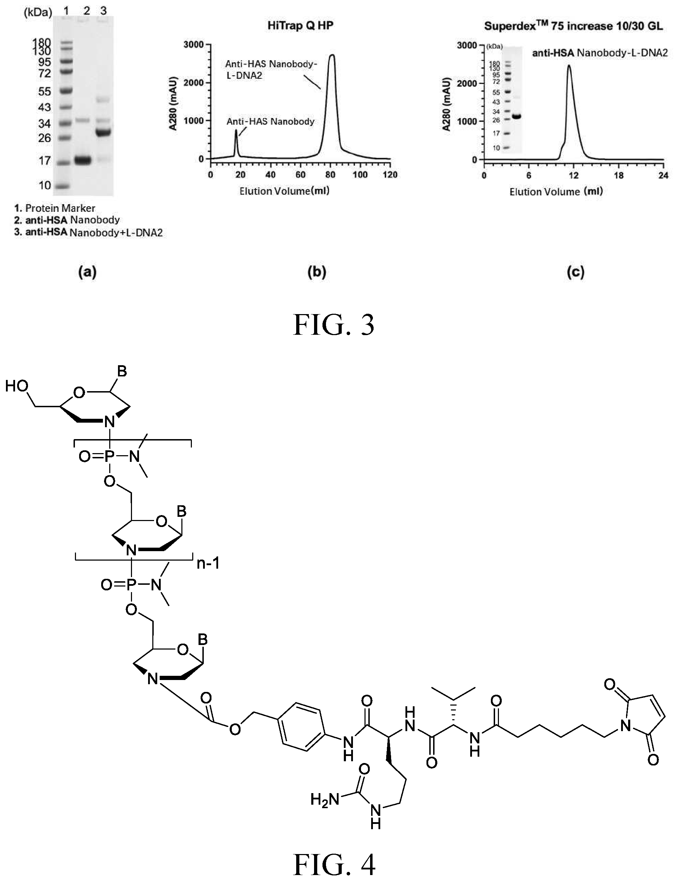

Figure 3 shows the conjugation and purification of the extended half-life module. (a) SDS-PAGE verification of the conjugation efficiency between the anti-HSA single-domain antibody (anti-HSA nanobody) and L-DNA2. Lane 1: Protein Marker; Lane 2: Anti-HSA single-domain antibody; Lane 3: Mixture of anti-HSA single-domain antibody and L-DNA2 after a 2-hour reaction at room temperature. (b) Free anti-HSA single-domain antibody was removed from the reaction mixture by anion-exchange chromatography. (c) Anti-HSA single-domain antibody-L-DNA2 with a purity greater than 95% was simultaneously obtained using molecular sieve displacement of the sample buffer.
Figure 4 shows the molecular structure of the antisense nucleic acid PMO.
Figure 5 shows the agarose gel verification of *in vitro* skipping of DMD exons 22 and 23.
Figure 6 shows the agarose gel analysis of the conjugation efficiency between the antisense nucleic acid PMO and the nucleic acid backbone L-DNA. Lanes 1 and 4: Molecular Weight Marker; Lane 2: L-DNA3; Lane 3: mixture of L-DNA3 and EXON22 PMO after 2-4 hours of reaction. Lane 5: L-DNA4; Lane 6: Mixture of L-DNA4 and EXON23 PMO after 2-4 hours of reaction.
Figure 7 shows the purification of PMO-(L-DNA)-PMO. (a) Free PMO was removed from the reaction mixture by anion-exchange chromatography. (b) The PMO-(L-DNA) conjugated to the preceding PMO molecule was removed from the reaction mixture by hydrophobic interaction chromatography. (c) Agarose gel verification of the purity of PMO-(L-DNA)-PMO. Lane 1: EXON22-(L-DNA3)-EXON22; Lane 2: EXON23-(L-DNA4)-EXON23; Lane 3: EXON23-(L-DNA3)-EXON23;
Figure 8 shows the schematic diagrams of MAOC molecules. (a) MAOC-001; (b) MAOC-002.
Figure 9 shows the agarose gel analysis of the purity of MAOC assembled samples. (a) SDS-PAGE confirms that MAOC-001 has a purity greater than 90%. (b) SDS-PAGE confirms that MAOC-002 has a purity greater than 90%. (c) 2% TAE agarose gel analysis of the sample purity before and after assembly of MAOC-002. Lane 1: Molecular weight marker; Lane 2: Anti-TfR1 single-domain antibody-L-DNA1; Lane 3: Anti-HSA single-domain antibody-L-DNA2; Lane 4: EXON22-(L-DNA3)-EXON22; Lane 5: EXON23-(L-DNA4)-EXON23; Lane 6: MAOC-002.
Figure 10 shows a schematic diagram of the ELISA experimental protocol.
Figure 11 shows ELISA analysis of the binding activity of the assembled MAOC-001 to human TfR1 protein and nucleic acid probes.
Figure 12 shows the body weight change curve of hTfR1 humanized mice treated with MAOC-001.
Figure 13 shows the establishment of an ELISA method for detecting PMO concentrations in serum and tissue samples. (a) Schematic diagram of the ELISA experimental protocol. (b) Double logarithmic linear fitting standard curve for EXON23 PMO sample. (c) Double logarithmic linear fitting standard curve for MAOC-001 sample.
Figure 14 shows the concentration-time curve of EXON23 PMO in the serum of mice treated with MAOC-001.
Figure 15 shows the levels of EXON23 PMO in various muscle tissues of mice treated with MAOC-001.
Figure 16 shows the agarose gel analysis of DMD exon 23 skipping in various muscle tissues of mice treated with MAOC-001.
Figure 17 shows a bar chart of DMD exon 23 skipping in various muscle tissues of mice treated with MAOC-001.
Figure 18 shows the 3% TAE agarose gel analysis of PCR product fragments for simultaneous skipping of DMD exons 22 and 23 *in vitro.*
Figure 19 shows the sequencing results of the PCR product fragments for skipping of DMD exons 22 and 23.
Figure 20 shows body weight changes in hTfR1 humanized mice treated with MAOC-002.
Figure 21 shows the levels of EXON22 PMO in various muscle tissues of mice treated with MAOC-002.
Figure 22 shows the levels of EXON23 PMO in various muscle tissues of mice treated with MAOC-002.
Figure 23 shows the agarose gel analysis of the double exon skipping of DMD exon 22 and 23 in various muscle tissues of mice treated with MAOC-002.
Figure 24 shows a bar chart of the double exon skipping of DMD exon 22 and 23 in various muscle tissues of mice treated with MAOC-002.
Figure 25 shows a schematic diagram of the complementary nucleic acid backbone composite structure.

### DETAILED DESCRIPTION

Through extensive and intensive research, the inventors have, for the first time, developed a polypeptide-oligonucleotide complex and a use thereof. Based on a complementary nucleic acid backbone complex structure, the present invention links a polypeptide element with a targeting function to an oligonucleotide with a pharmacodynamics function, resulting in a multifunctional complex. The complex of the present invention exhibits precise targeting and excellent pharmacokinetic properties, and meanwhile has a high oligonucleotide-to-antibody ratio, enabling precise and highly efficient gene therapy. The present invention is completed on this basis.

The complexes of the present invention are simple and convenient to prepare. According to requirements, on the basis of preparing polypeptide monomers, oligonucleotide monomers and pure backbone monomers containing backbone single-stranded nucleic acids, flexible assembly is carried out through the paired structures of backbone single-stranded nucleic acids, so as to obtain multifunctional complexes with a required oligonucleotide/antibody ratio.

### Term

Unless otherwise defined, all technical and scientific terms used herein have the same meanings as are commonly understood by those skilled in the art to which the present invention pertains. As used herein, when referring to specifically listed numerical values, the term "about" means that the value may vary by no more than 1% from the listed value. For example, as used herein, the expression "about 100" includes 99, 101, and all values therebetween (e.g., 99.1, 99.2, 99.3, 99.4, etc.).

As used herein, the terms "complementary nucleic acid backbone composite structure", "paired nucleotide backbone structure", "PNSC (paired nucleotide backbone complex structure)" are used interchangeably and refer to a complex paired structure formed by two or more (e.g., 2, 3, 4, 5, 6) paired nucleotide backbone monomers, wherein the backbone nucleic acid single strand of each monomer forms a paired double strand with the backbone nucleic acid single strand of another monomer through complementary base pairing, thereby forming a paired nucleotide backbone complex structure. The paired nucleic acid backbone composite structures of the present invention may be dimers, trimers, tetramers, pentamers, hexamers, heptamers, or octamers. Representative structures include, but are not limited to, those shown in Figure 25.

As used herein, the terms "monomer having a complementary nucleic acid backbone" and "complementary nucleic acid backbone monomer" are used interchangeably and refer to a monomer that has a nucleic acid single strand, and wherein the nucleic acid single strand includes a backbone nucleic acid pairing region for forming a complementary nucleic acid backbone.

As used herein, the term "backbone nucleic acid single strand" refers to a nucleic acid single strand having a backbone nucleic acid pairing region. The backbone nucleic acid single strand may be present in polypeptide monomers, oligonucleotide monomers, and pure backbone monomers.

### Pharmacodynamic Element

In the present invention, the pharmacodynamic element is an oligonucleotide-based drug. Typically, such oligonucleotide-based drugs include, but are not limited to, antisense oligonucleotides (AONs), small interfering RNAs (siRNAs), and microRNAs.

In some embodiments, the pharmacodynamic element may be natural nucleotides and/or chemically modified nucleotides. Chemical modification is one of the most effective methods for enhancing the efficacy of oligonucleotide drugs, it helps antisense oligonucleotides resist degradation by endogenous nucleases, improves the stability of oligonucleotides in plasma, tissues, and cells, and can also increase their affinity for target nucleic acids, thereby achieving higher antisense activity.

Chemical modifications include modifications to the nucleic acid backbone, modifications to the bases, modifications to the ribose glycosyl, and the use of nucleic acid analogs. The term "nucleic acid analogs" refers to groups capable of replacing natural nucleotides in nucleic acids, including but not limited to bridged nucleotides (BNAs), acyclic nucleotides, or isonucleotides. BNAs are a type of nucleotide in which the ribose is constrained in the 3'-endo conformation by a bridge between the 2' and 4' carbon atoms. BNAs include locked nucleic acids (LNAs), ENAs, cETs, or other types of bicyclic nucleotides. BNAs increase the stability of oligonucleotides against nucleases and their affinity for target RNA. Acyclic nucleotides include unlocked nucleic acids (UNAs) or glycerol nucleic acids (GNAs). Isonucleotides include peptide nucleic acids (PNAs) or phosphorodiamidate morpholino (PMOs).

### L-Nucleic Acid

L-nucleic acid refers to the mirror-image counterparts of naturally occurring D-nucleic acid (D-nucleic acid) and can be classified into L-DNA and L-RNA. The levogyration (chiral) center is primarily located in the deoxyribose or ribose moiety of nucleic acids and exhibits a mirror-image inversion. Consequently, L-nucleic acids cannot be degraded by the nucleases (such as exonucleases and endonucleases) that are ubiquitous in plasma.

### Polypeptide Element

In the present invention, the polypeptide element may specifically target cell surface molecules and induce endocytosis. Typically, the polypeptide element comprises a humanized antibody or binding fragment thereof, a chimeric antibody or binding fragment thereof, a monoclonal antibody or binding fragment thereof, a monovalent Fab', a bivalent Fab2, a single-chain variable fragment (scFv), a bispecific antibody, a mini-antibody, a single-domain antibody, a single-domain antibody (sdAb), or a camelid antibody or binding fragment thereof.

In another preferred embodiment of the present invention, the polypeptide element further comprises a protein or polypeptide with extended half-life (polypeptide element with extended half-life). Representative examples include, but are not limited to: anti-human serum albumin (anti-HSA) antibodies, Fc elements, or combinations thereof.

In a preferred embodiment, the extended half-life module employs an anti-HSA single-domain antibody, which, by binding to the HSA protein in serum, can extend the half-life of macromolecular drugs through the FcRn-mediated protein recycling mechanism.

### Targeting Polypeptide Element

As used herein, the terms "polypeptide element having a targeting function" and "targeting polypeptide element" are used interchangeably. Representative targeting polypeptide elements include (but are not limited to): antibodies, ligands, and nucleic acid aptamers against cell surface receptors, or combinations thereof.

A preferred targeting polypeptide element is an antibody or active fragment thereof, a ligand or active fragment thereof, or a combination thereof.

### Transferrin Receptor 1

Transferrin receptor 1 (TfR1) is a membrane protein widely expressed in nearly all cell and tissue types in the human body, including muscle tissue and cells; TfR1 is widely expressed in the heart, liver, kidney, muscle, and other tissues.

### Pharmaceutical Composition

The present invention also provides a composition. In a preferred embodiment, the composition is a pharmaceutical composition comprising the aforementioned antibody or active fragment thereof or fusion protein thereof, and a pharmaceutically acceptable carrier. Typically, these substances may be formulated in a non-toxic, inert, and pharmaceutically acceptable aqueous carrier medium, in which the pH is typically about 5-8, more preferably about 6-8, although the pH may vary depending on the nature of the substances being formulated and the condition to be treated. The prepared pharmaceutical composition may be administered by conventional routes, including (but not limited to): oral, inhalation, intratumoral, intraperitoneal, intravenous, or topical administration.

The pharmaceutical composition of the present invention can be used directly for treatment (e.g., antitumor therapy), thereby extending the half-life of the drug; furthermore, it can be used in combination with other therapeutic agents.

The pharmaceutical composition of the present invention contains a safe and effective amount (e.g., 0.001-99 wt%, preferably 0.01-90 wt%, more preferably 0.1-80 wt%) of the aforementioned monoclonal antibody (or its conjugate) of the present invention, as well as a pharmaceutically acceptable carrier or excipient. Such carriers include (but are not limited to): saline, buffers, glucose, water, glycerol, ethanol, and combinations thereof. The pharmaceutical formulation should be tailored to the route of administration. The pharmaceutical compositions of the present invention can be formulated as injections, for example, prepared by conventional methods using saline or an aqueous solution containing glucose and other excipients. Pharmaceutical compositions such as injections and solutions should be manufactured under sterile conditions. The dosage of the active ingredient is a therapeutically effective dose, for example, about 1 microgram/kilogram of body weight to about 10 milligrams/kilogram of body weight per day. Furthermore, the polypeptides of the present invention may also be used in combination with other therapeutic agents.

When using the pharmaceutical composition, a safe and effective dose of the immunoconjugate is administered to mammals, wherein the safe and effective dose is typically at least about 10 micrograms per kilogram of body weight and, in most cases, does not exceed about 8 milligrams per kilogram of body weight; preferably, the dose ranges from about 10 micrograms per kilogram of body weight to about 1 milligram per kilogram of body weight. Of course, the specific dose should also take into account factors such as the route of administration and the patient's health status, all of which fall within the scope of a skilled physician's expertise.

### Duchenne Muscular Dystrophy

Duchenne muscular dystrophy (DMD) is a hereditary muscle-wasting disease. The disease is caused by a mutation in the dystrophin gene, resulting in the absence of the dystrophin protein, and is primarily characterized by progressive muscle atrophy and weakness. Patients begin to exhibit symptoms of muscle weakness at the ages of 2 to 5, gradually lose the ability to walk at ages of 9 to 12, and ultimately, the disease affects all smooth muscles, as well as the heart and respiratory muscles, leading to death from respiratory failure or cardiac dysfunction. The average life expectancy for patients with this disease is about 30 years. Current treatments for this disease include glucocorticoid injections and gene therapy, however, these methods can only slow the progression of the disease and cannot cure DMD.

### The main advantages of this invention are as follows:

(1) Compared to traditional oligonucleotide drugs, the polypeptide-oligonucleotide multimer complex of the present invention exhibits precise targeting.
(2) Compared to traditional oligonucleotide drugs, the polypeptide-oligonucleotide multimer complex of the present invention can cross the blood-brain barrier and exhibits superior pharmacokinetic properties.
(3) Compared to conventional AOC drugs, the polypeptide-oligonucleotide multimer complex of the present invention can load multiple oligonucleotide pharmacodynamic elements through a complementary nucleic acid backbone composite structure using an extremely simple and rapid linking method, thereby achieving a high oligonucleotide-to-antibody ratio.
(4) The polypeptide-oligonucleotide multimer complex of the present invention is simple and flexible to assemble and can simultaneously load different types of oligonucleotide pharmacodynamic elements.
(5) The polypeptide-oligonucleotide multimer complex of the present invention is simple and flexible to assemble, can simultaneously target multiple targets, and enables more precise targeting.

The present invention will be further described below with reference to specific examples. It should be understood that these examples are intended to illustrate the present invention and are not intended to limit its scope. Experimental methods in the following examples for which specific conditions are not specified are generally performed under conventional conditions, such as those described in Sambrook et al., Molecular Cloning: A Laboratory Manual (New York: Cold Spring Harbor Laboratory Press, 1989), or as recommended by the manufacturer. Unless otherwise specified, percentages and parts refer to weight percentages and weight parts.

### Example 1: Nucleic Acid Backbone and MAOC Molecular Design

The examples of the present invention employ a tetrameric L-DNA nucleic acid backbone, with the sequences of the four nucleic acid single strands as follows (from 5' to 3'):
Strand 1 (L-DNA1): SEQ ID NO: 121
   5'-AGGCGATCACAATCCAAATGAGCGTGTTACGG -3'
Strand 2 (L-DNA2): SEQ ID NO: 122
   5'-ACCGTAACACGCTCAAAACCGAAGTGCCAATT-3'
Strand 3 (L-DNA3): SEQ ID NO: 123
   5'-AAATTGGCACTTCGGAAAACTATGCGGCTGCT-3'
Strand 4 (L-DNA4): SEQ ID NO: 124
   5'-AAGCAGCCGCATAGTAAAGGATTGTGATCGCC-3'

Wherein, the 5' ends of Strands 1 and 2 are modified with NH₂ groups, while the 5' and 3' ends of Strands 3 and 4 are modified with disulfide bonds, respectively.

MAOC molecules primarily consist of a targeting module composed of a single-domain antibody-nucleic acid backbone conjugate and a pharmacodynamic module composed of an oligonucleotide-nucleic acid backbone-oligonucleotide conjugate, supplemented by an extended half-life module. A schematic diagram of some molecules is shown in Figure 1.

### Example 2: Preparation of the Targeting Module: Anti-TfR1 Single-Domain Antibody-L-DNA1

A cysteine mutation was introduced at the carboxy-terminus of the single-domain antibody for conjugating to a nucleic acid backbone. The gene sequence of the anti-TfR1 single-domain antibody was optimized for yeast-preferred codons and then subcloned into the pPICZ alpha A plasmid. The amino acid sequence of the anti-TfR1 single-domain antibody is SEQ ID NO: 361. A His tag was added to the N-terminus of the single-domain antibody to facilitate purification.
SEQ ID NO: 361, amino acid sequence of the anti-TfR1 single-domain antibody mutant:

HHHHHHEVQLVESGGGLVQPGGSLRLSCAASGFTFSSKTMSWLRQAPGKGLEWVSSI
NSGGSGTDYADSVKGRFTISRDNAKNTLYLQMNSLRAEDTAVYYCAINGPNYYRESTRGQ
GTLVTVSSGSC

The plasmid was linearized, and then electroporated into Pichia pastoris strain X33, and high-copy strains expressing the target gene were selected using Zeocin concentration gradient YPD agar plates. The monoclonal strain was cultured in GMGY medium at 30°C and 250 rpm. After sufficient cell mass was obtained, the target single-domain antibody was induced for secretory expression in GMMY medium at 20°C and 250 rpm, with 1% methanol added every 24 hours. The strains subjected to high-copy screening can achieve a single-domain antibody expression yield of 40-200 mg/L in laboratory-scale glass flasks. The single-domain antibodies from the culture supernatant were purified using a His-tag affinity column.

The NH₂ group at the 5' end of L-DNA1 was modified using the SMCC linker to introduce a maleimide active group at the 5' end of L-DNA1 . The cysteine at the carboxy-terminus of the single-domain antibody was reduced using a reducing agent. The reduced single-domain antibody was mixed with 1fold to 2-fold molar excess of SMCC-L-DNA1 single-strands, placed at room temperature for 2 hours of conjugation reaction, and the conjugation efficiency was verified via SDS-PAGE (Figure 2(a)).

Unreacted SMCC-L-DNA1 single strands were removed using a His-tag affinity column, and the single-domain antibody and the single-domain antibody-L-DNA mixture were collected. The buffer was replaced with the loading buffer for the anion-exchange column. The single-domain antibody-L-DNA was further separated and purified using a HiTrap Q HP (Cytiva) to remove trace amounts of unreacted single-domain antibody (Figure 2(b)). The separation was achieved via gradient elution. The loading buffer was 20 mM Tris-HCl, 15 mM NaCl, pH 7.4, and the elution buffer was 20 mM Tris-HCl, 1 M NaCl, pH 7.4. Gradient elution was performed using 0-100% elution buffer, and the unreacted single-domain antibodies and the single-domain antibody-L-DNA were eluted successively. The single-domain antibody-L-DNA was collected, concentrated, and then elution buffer was replaced with PBS using Superdex^{™} 75 increase 10/300 GL (Cytiva). Ultimately the anti-TfR1 single-domain antibody-L-DNA1 with a purity greater than 95% was obtained (Figure 2(c)).

### Example 3: Preparation of the Extended Half-Life Module: Anti-HSA Single-Domain Antibody-L-DNA2

The single-domain antibody used in the extended half-life module is an anti-HSA single-domain antibody. By binding to the HSA protein in serum, this antibody can extend the half-life of macromolecular drugs through the FcRn-mediated protein recycling mechanism. The amino acid sequence of the anti-HSA single-domain antibody is SEQ ID NO: 362. A His tag was added to the N-terminus of the single-domain antibody to facilitate purification.
SEQ ID NO: 362, amino acid sequence of the anti-HSA single-domain antibody mutant:

HHHHHHEEVQLVESGGNLVQAGDSLTLSCEASGHLFSNYVLGWFRQRTGEEREYVASI
SRTGKDVRYSDSVKGRFTIYRDNTKNTVYLKMTRLEPEDTAVYLCAIYNGARGEYGDWGQ
GTQVTVSSGSC

The methods for the expression, purification, and conjugation of the anti-HSA single-domain antibody with L-DNA2 were the same as in Example 2. Following the conjugation and purification steps, anti-HSA single-domain antibody-L-DNA2 with a purity greater than 95% was obtained (Figure 3).

### Example 4: Synthesis of Antisense Oligonucleotide PMO Sequences

The present invention employs two PMO sequences: EXON22, which skips exon 22 of the mouse-derived DMD gene and has the sequence as shown in SEQ ID NO: 363; and EXON23, which skips exon 23 of the mouse-derived DMD gene and has the sequence as shown in SEQ ID NO: 364.
EXON22: SEQ ID NO: 363
   5'-ATGTCCACAGACCTGTAATT-3'
EXON23: SEQ ID NO: 364
   5'-GGCCAAACCTCGGCTTACCTGAAAT-3'

The 3' ends of the two PMOs were each modified with an MC-VC-PAB linker for L-DNA conjugation. The molecular formula is shown in Figure 4.

### Example 5: PMO-Induced DMD Exon Skipping in Mouse Myoblasts C2C12

Mouse myoblasts C2C12 cells were seeded at a density of 100,000 cells per well in a 12-well plate, and the cell plates were incubated overnight in an incubator at 37°C and 5% CO₂ using DMEM supplemented with 10% FBS. On the next day, the medium was replaced with differentiation medium consisting of DMEM + 2% horse serum (Procell, Cat#164215) + 1 µM insulin (Procell, Cat#PB180432), and the cell plates were placed in incubator for further incubation for 3-5 days, with medium refreshed every 2 days. 10-fold concentrated EXON22 PMO, EXON23 PMO and control Oligo were prepared, 100 µL was added to 900 µL fresh differentiation medium to incubate cells. After 24 hours of drug treatment, the medium was replaced with drug-free differentiation medium, then drug-free differentiation medium was replaced every day until cells were harvested after 72 hours of drug treatment. TRIzol reagent (Thermo, Cat#15596018) was used to lyse cells, total RNA was isolated via phenol/chloroform method, then M-MuLV First-Strand cDNA Synthesis Kit (Sangon, Cat#B532435) was used for reverse transcription into cDNA. 300 ng cDNA was taken to perform primary PCR reaction using Taq PCR Mix premix (Sangon, Cat#B639293), with primers Ex20F: 5'-CAGAATTCTGCCAATTGCTGAG-3' (SEQ ID NO: 365) and Ex26R: 5'-TTCTTCAGCTTGTGTCATCC-3' (SEQ ID NO: 366), and the reaction protocol was shown in Table 1.

**Table 1: Primary PCR Protocol**

| | | | |
|---|---|---|---|
| Hot Start | 95°C | 2 minutes | 1 cycle |
| Denaturation | 95°C | 0.5 minutes | |
| Primer annealing | 53°C | 1 minute | 35 cycles |
| Primer extension | 72°C | 2 minutes | |
| Final extension | 72°C | 10 minutes | 1 cycle |
| End point | 4°C | - | - |

1 µL of the primary PCR reaction product was taken for use in a 50 µL nested PCR reaction. The primers used were Ex20F2: 5'-ACCCAGTCTACCACCCTATC-3' (SEQ ID NO: 367) and Ex25R: 5'-CTCTTTATCTTCTGCCCACCTT-3' (SEQ ID NO: 368). The reaction protocol was shown in Table 2.

**Table 2: Nested PCR Protocol**

| | | | |
|---|---|---|---|
| Hot Start | 95°C | 2 minutes | 1 cycle |
| Denaturation | 95°C | 0.5 minutes | |
| Primer annealing | 50°C | 1 minute | 25 cycles |
| Primer extension | 72°C | 1 minute | |
| Final extension | 72°C | 10 minutes | 1 cycle |
| End point | 4°C | - | - |

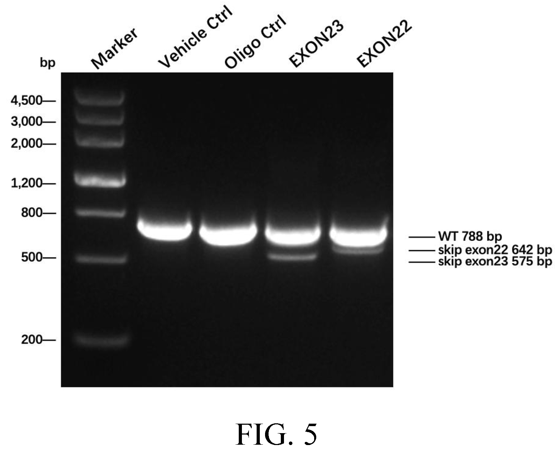

The PCR reaction products were analyzed using a 1% TAE agarose gel. The product with wild-type (WT) DMD was 788 bp in length, the product with DMD exon 22 skipping (skip exon 22) was 642 bp, and the product with DMD exon 23 skipping (skip exon 23) was 575 bp. No exon skipping was observed in the vehicle control (Vehicle Ctrl) or the oligo control (Oligo Ctrl) groups (Figure 5).

### Example 6: Preparation of the Pharmacodynamic Module: PMO-(L-DNA3/4)-PMO

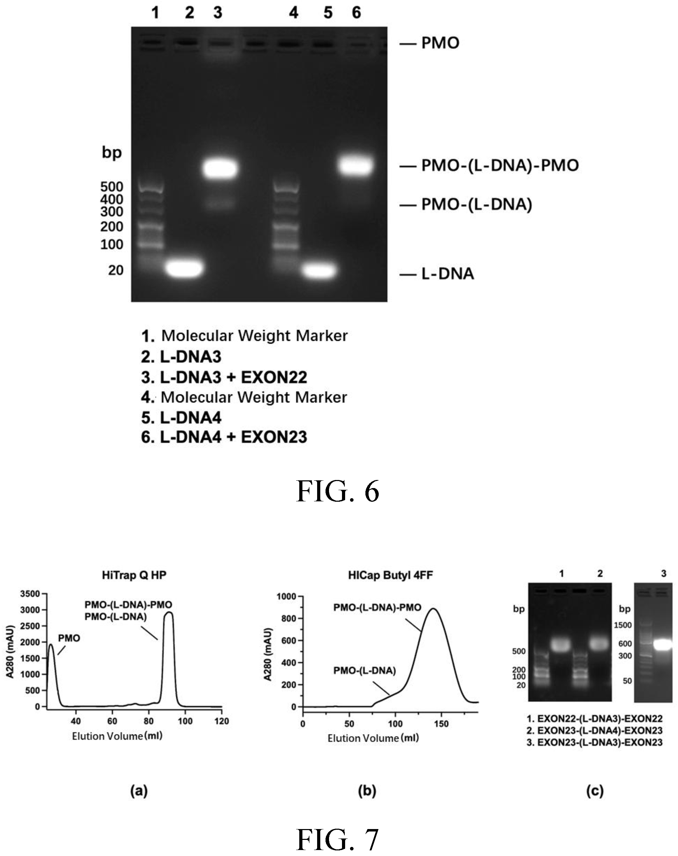

The pharmacodynamic element PMO-(L-DNA)-PMO was prepared by conjugating two PMO chains to the 3' and 5' ends of L-DNA, respectively. First, the disulfide bonds modified at the 3' and 5' ends of L-DNA were reduced to expose a thiol group at each end, respectively. Next, PMO modified with MC-VC-PAB linker at 3' end was added at 5-fold molar amount, mixed rapidly and incubated at 60°C for 2-4 hours. After the reaction was completed, the conjugation efficiency was assessed using a 2% TAE agarose gel. The experimental results showed that the proportion of L-DNA 1 and L-DNA2 conjugated with EXON22 and EXON23 at both 3' and 5' ends was over 95%, only a small number of L-DNA molecules were conjugated to a single PMO, and there was virtually no residual free L-DNA (Figure 6).

The purification of PMO-(L-DNA)-PMO consisted of two main steps: 1) Taking advantage of the uncharged nature of PMO, free PMO was removed using a HiTrap Q HP (Cytiva) (Figure 7(a)); 2) Taking advantage of the difference in hydrophobicity between PMO-(L-DNA)-PMO and PMO-(L-DNA), the two were separated using a HICap Butyl 4FF column (Figure 7(b)). Analysis via a 2% TAE agarose gel showed that the purity of the final PMO-(L-DNA)-PMO sample was greater than 98% (Figure 7(c)).

### Example 7: Self-Assembly of MAOC Drugs

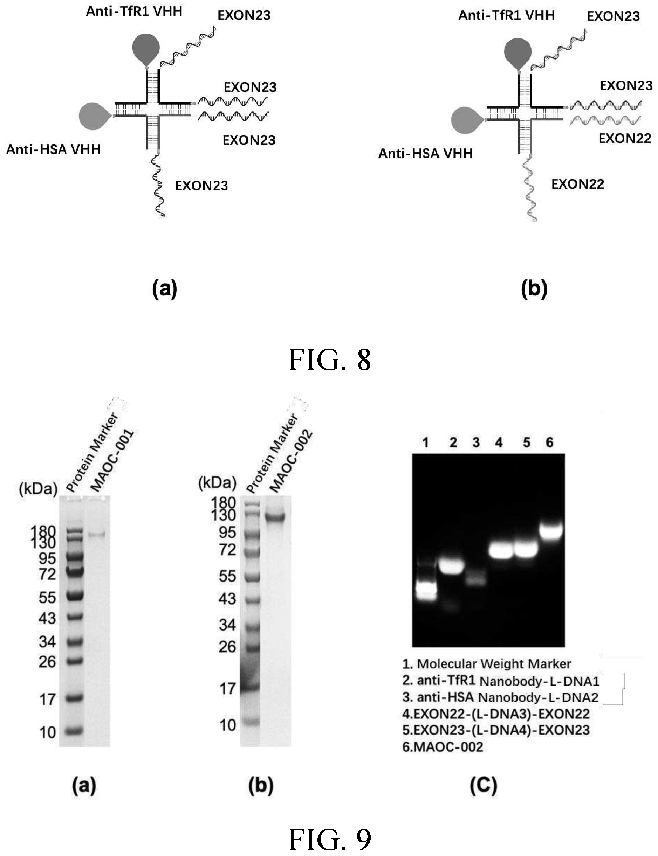

This example illustrates the self-assembly of MAOC molecules using two MAOC molecules, MAOC-001 and MAOC-002, as examples. The MAOC-001 molecule was conjugated with four EXON23 PMOs (Fig. 8(a)), allowing a single antibody to simultaneously mediate the delivery of four oligonucleotide molecules; while the MAOC-002 molecule was conjugated with two types of PMOs (Fig. 8(b)), enabling dual exon skipping.

The self-assembly process of the MAOC-001 drug was as follows:
The concentrations of the anti-TfR1 single-domain antibody (L-DNA1), the anti-HSA single-domain antibody (L-DNA2), EXON23-(L-DNA3)-EXON23, and EXON23-(L-DNA4)-EXON23 were determined using a NanoDrop One (Thermo), respectively. An appropriate amount of each of the above components was preheated at 37°C for 5 minutes, then mixed at a 1:1 molar ratio at 37°C and incubated for 1 minute, thereby completing the self-assembly of the MAOC-001 drug. The purity of the assembled sample was determined by SDS-PAGE, and the validation results showed that the purity of the assembled sample was greater than 90% (Figure 9(a)).

The self-assembly process for the MAOC-002 drug was as follows:
The concentrations of the anti-TfR1 single-domain antibody-L-DNA1, anti-HSA single-domain antibody-L-DNA2, EXON22-(L-DNA3)-EXON22, and EXON23-(L-DNA4)-EXON23 were determined using a NanoDrop One (Thermo), respectively. An appropriate amount of each of the above components was preheated at 37°C for 5 minutes, then mixed at a 1:1 molar ratio at 37°C and incubated for 1 minute, thereby completing the self-assembly of the MAOC-002 drug. The purity of the assembled samples was determined using SDS-PAGE and a 2% TAE agarose gel, respectively, and the validation results showed that the purity of the assembled samples was greater than 90% (Fig. 9(b, c)).

### Example 8: Validation of the Targeting Activity of MAOC Drugs

The binding activity of the assembled MAOC-001 and MAOC-002 to human TfR1 protein and nucleic acid probes, respectively, was analyzed using an Enzyme-Linked Immunosorbent Assay (ELISA). Two PTO/DNA probes, which were complementary to EXON22 PMO and EXON23 PMO respectively, were synthesized, and the 5' end of each probe was labeled with digoxigenin, and the 3' end with biotin.
EXON22 PTO/DNA probe: SEQ ID NO: 369
   5'-digoxigenin-**AATTAC**AGGTCTGT**GGACAT**-biotin-3'
EXON23 PTO/DNA probe: SEQ ID NO: 370
   5'-digoxigenin-**ATTTCAGGT**AAGCCGAGGTTTGGCC-biotin-3'
wherein, the nucleotides marked in bold were subjected to thiol modification.

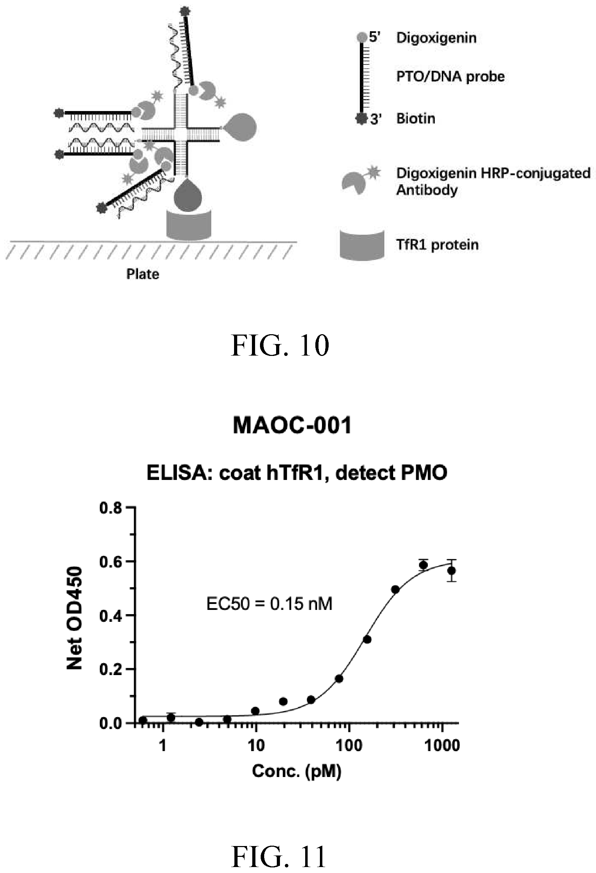

A schematic diagram of the ELISA experimental protocol is shown in Figure 10. MAOC molecules were captured through the binding of human TfR1 protein to the anti-TfR1 single-domain antibody on the MAOC. Subsequently, PMO on the MAOC molecules was detected using a PTO/DNA probe complementary to PMO, thereby verifying the activity of the targeting module and the pharmacodynamic module on the MAOC molecules, as well as the integrity of the MAOC molecules. The experimental procedure was as follows:
Human TfR1-His (ACRO, Cat#CD1-H5243) antigen solution was prepared with PBS to a final concentration of 1 µg/mL, 25 µL per well was added to a 384-well microplate, and it was coated at 4 °C overnight. Washing was carried out 3 times with PBST (PBS + 0.05% Tween 20), 50 µL per well blocking solution (PBST + 3% BSA) was added, and it was blocked at room temperature for 1 hour. Washing was carried out once with PBST. MAOC-001 or MAOC-002 was diluted gradiently with sample dilution buffer (10 mM Tris, 1 mM EDTA, 0.1% (v/v) Triton X-100, pH 8.0); EXON22 PTO/DNA or EXON23 PTO/DNA capture probe was diluted gradiently with probe dilution buffer (10 mM Tris, 1 mM EDTA, 1 M NaCl, 0.1% (v/v) Triton X-100, pH 8.0). Equal volumes of the above diluted MAOC drug and capture probe were mixed and incubated at 37 °C for 30 minutes. The mixed solution was transferred to a 384-well microplate pre-coated with hTfR1 at 25 µL per well with replicate wells set, and incubated at 37 °C for 30 minutes. Micrococcal nuclease (CST, Cat#10011) solution (diluted 1:5000) was prepared with nuclease reaction buffer (50 mM Tris, 5 mM CaCl₂, 200 mM NaCl, 0.1 mg/mL BSA, pH 8.2), and 25 µL per well was added to the microplate. Incubation was performed at 37 °C for 1 hour to allow Micrococcal nuclease to cleave the probes that were unbound to PMO. Washing was carried out 3 times with PBST, 25 µL per well horseradish peroxidase-labeled anti-digoxigenin antibody (R&D Systems, Cat#HAM7520) solution diluted at 1:5000 was added, and incubated at 37 °C for 30 minutes. Washing was carried out 3 times with PBST, the plate was patted dry, 25 µL per well chromogenic substrate TMB solution (Beyotime, Cat#P0209) was added for color development at room temperature for 5 to 30 minutes, then 25 µL per well color termination solution (Beyotime, Cat#P0215) was added. A multi-mode microplate reader (Molecular Devices, SpectraMax i3x) was used to measure the absorbance value at 450 nm of each well. The 4-parameter sigmoidal curve equation of GraphPad Prism 9 software was used for fitting to calculate EC50 value for binding. Experimental results presented a standard S-shaped curve, as shown in Figure 11, with an EC50 value of 0.15 nM, indicating that MAOC-001 could bind human TfR1 protein and PTO/DNA probes simultaneously, possessed an intact molecular structure, and had strong binding capacity.

### Example 9: Serum Pharmacokinetic Analysis of MAOC-001 in hTfR1 Humanized Mice

For in vivo pharmacodynamic and pharmacokinetic studies, hTfR1-humanized C57BL/6 mice weighing 16-24 g and aged 6-8 years were selected. A single injection of 20 mg/kg EXON23 PMO and MAOC-001 (at a molar dose of 1/4 that of EXON23 PMO) were administered respectively, with a PBS group serving as the control. Serum and tissue samples were collected at various time points to further analyze the *in vivo* pharmacokinetics, tissue distribution, and *in vivo* and *in vitro* exon skipping of the drug in mice. Seventeen mice were grouped by body weight and randomly divided into three groups of 5-6 mice each. Detailed dosing information is shown in Table 3:

**Table 3 Grouping and Administration**

| **Group** | **Test Substance** | **Number of Animals (mice)** | **Dose (mg/kg PMO)** | **Administration Route** | **Administration Frequency ^{a}** |
|---|---|---|---|---|---|
| G1 | PBS | 5 | - | i.v. | Single dose |
| G2 | EXON23 | 6 | 20 | i.v. | Single dose |
| G3 | MAOC-001 | 6 | 20 | i.v. | Single dose |

| | | | | | |
|---|---|---|---|---|---|
| Note: a: A single dose refers to a single administration on the day of grouping. | | | | | |

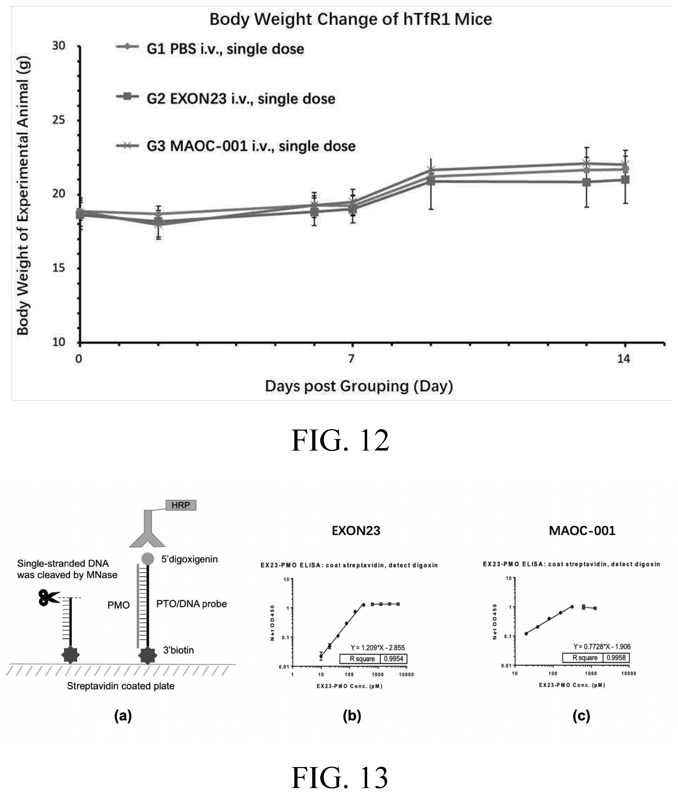

All mice underwent clinical observation once daily, which included, but was not limited to, assessment of the animals' condition and feeding behavior. Body weight was measured twice weekly, and monitoring data showed that the body weights of all mice fluctuated within the normal range throughout the experiment, indicating that the mice tolerated the drug well at the tested doses (Figure 12). At the end of the experiment or upon reaching a humane endpoint, the animals were euthanized using an overdose of CO₂.

Blood samples were collected from the treatment group 5 min, 2 h, 4 h, 8 h, 1 day, 4 days, 7 days, and 14 days after administration; blood samples were also collected from mice in the untreated group as controls. Following blood collection, serum was rapidly separated and stored at -80°C.

The ELISA method was used to detect PMO concentrations in serum samples, as illustrated in the schematic diagram (Figure 13(a)). Streptavidin (ACRO, Cat#STN-N5116) was used to capture biotin-labeled PTO/DNA probes, which in turn captured PMO in the serum through base-pairing. Micrococcal nuclease was then used to cleave the probes that were unbound to PMO. Washing was performed to remove free digoxigenin, and then the PMO content in the serum was detected using an anti-digoxigenin antibody (R&D Systems, Cat#HAM7520) diluted 1:5000. The standard curve was prepared using a 2-fold gradient dilution method, starting at 2000 pM, with 11 concentration levels. All samples were tested in duplicate, and secondary antibody control and blank mouse blood control groups were set. Net OD450 = T_{OD}- C_{OD}, where T_{OD} is the actual OD450 measurement of each serum sample, and C_{OD} is the average OD450 measurement of the secondary antibody control group. A standard curve was fitted using the double logarithmic method, and the detection range of the EXON23 samples was 10 pM-313 pM, with R² ≥ 0.99 (Figure 13(b)); the detection range of the MAOC-001 samples was 20 pM-313 pM, with R² ≥ 0.99 (Figure 13(c)).

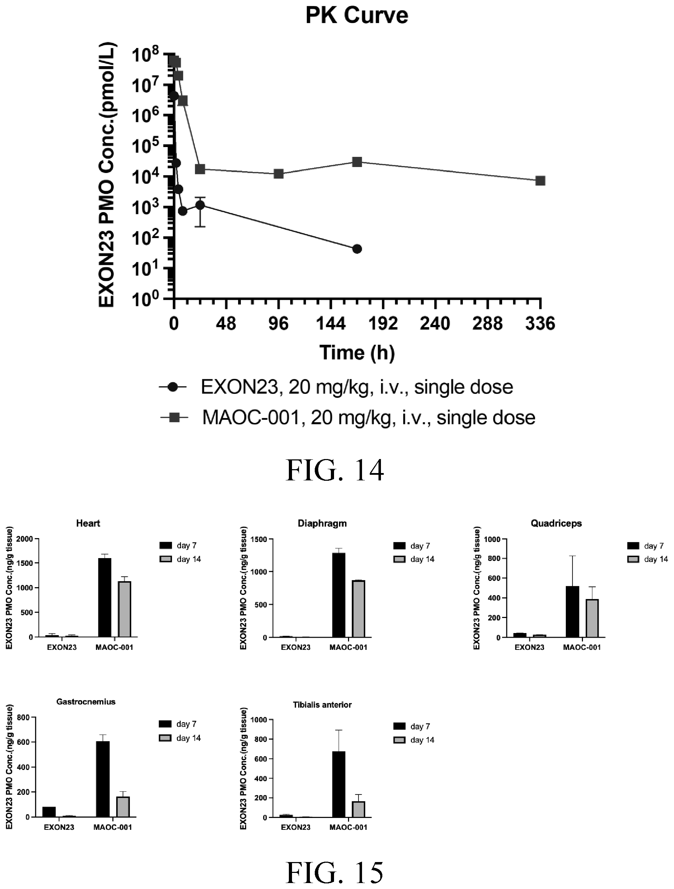

Each serum sample was diluted to concentrations within the detection range, and the corresponding Net OD450 values were measured. Subsequently, the corresponding serum drug concentrations were calculated based on the standard curve, and drug concentration-time curves were plotted (Figure 14). The experimental results indicated that, at equal PMO dosages, the serum PMO concentrations in the MAOC-001 group were higher than those in the EXON23 PMO group at all blood sampling time points, and compared to EXON23 PMO, MAOC-001 exhibited higher bioavailability.

### Example 10: Analysis of oligonucleotide delivery efficiency of MAOC-001 in the muscle tissue of hTfR1 humanized mice

At two time points, 7 and 14 days after administration, 2-3 mice were selected from each group. After anesthesia, the mice were perfused with pre-chilled PBS. After perfusion was completed, tissue samples of heart, diaphragm, bilateral quadriceps, bilateral gastrocnemius, bilateral soleus, and bilateral tibialis anterior muscles were rapidly collected. After collection, each tissue sample was divided into two portions: the first portion was weighed, flash-frozen in liquid nitrogen, and stored at -80°C for analysis of PMO content in the tissue; the second portion was weighed and immersed in a solution of RNA Solid (Servicebio, Cat#G3019) with a volume 10 times that of the tissue, it was first incubated at 4°C overnight and then stored at -80°C for total RNA extraction and exon skipping analysis.

After the tissue samples were ground, the PMO concentration in the tissue was measured using the ELISA method, following the same procedure as in Example 9. A bar chart was plotted based on the experimental data (Figure 15). The experimental results showed that in all tissue samples, including the heart, diaphragm, quadriceps, gastrocnemius, and tibialis anterior, the EXON23 PMO levels in the MAOC-001 treatment group (G3) were significantly higher than those in the EXON23 PMO treatment group (G2). Therefore, the oligonucleotide delivery efficiency of MAOC-001 in mice was significantly higher than that of the oligonucleotide itself.

### Example 11: Analysis of the DMD exon skipping efficacy of MAOC-001 in hTfR1 humanized mice

After each tissue sample was ground, total RNA was extracted and exon skipping analysis was performed. TRIzol reagent (Thermo, Cat#15596018) was used to lyse the ground tissue samples, and total RNA was isolated using the phenol/chloroform method. 10-200 ng of total RNA was taken, and reverse transcription and primary PCR were performed simultaneously using the HiScript II One Step RT-PCR Kit (Vazyme, Cat#P611). The primers used were Ex20F2: 5'-ACCCAGTCTACCACCCTATC-3' and Ex25R: 5'-CTCTTTATCTTCTGCCCACCTT-3', with the reaction protocol shown in Table 4.

**Table 4: Reverse Transcription and Primary PCR Protocols**

| | | | |
|---|---|---|---|
| Reverse Transcription | 50°C | 30 minutes | 1 cycle |
| Hot start | 95°C | 2 minutes | 1 cycle |
| Cool-down PCR | 64-50°C | 1 minute | 1 degree decrease per cycle |
| Denaturation | 95°C | 0.5 minutes | |
| Primer annealing | 50°C | 1 minute | 25 cycles |
| Primer extension | 72°C | 2 minutes | |
| Final extension | 72°C | 10 minutes | 1 cycle |
| End point | 4°C | - | - |

1 µL of the primary PCR reaction product was taken for use in a 50 µL nested PCR reaction system. The primers used were Ex22F: 5'-TGAGTAGCATCAGGACGTGG-3' (SEQ ID NO: 371) and Ex24R: 5'-GCAGGCCATTCCTCTTTCAG-3' (SEQ ID NO: 372). The reaction protocol is shown in Table 5.

**Table 5: Nested PCR Protocol**

| | | | |
|---|---|---|---|
| Hot Start | 95°C | 2 minutes | 1 cycle |
| Denaturation | 95°C | 0.5 minutes | |
| Primer annealing | 48°C | 1 minute | 25 cycles |
| Primer extension | 72°C | 1 minute | |
| Final extension | 72°C | 10 minutes | 1 cycle |
| End point | 4°C | - | - |

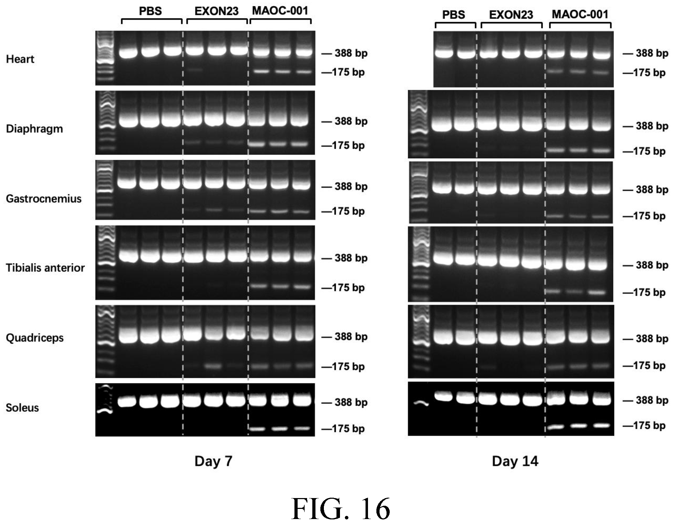

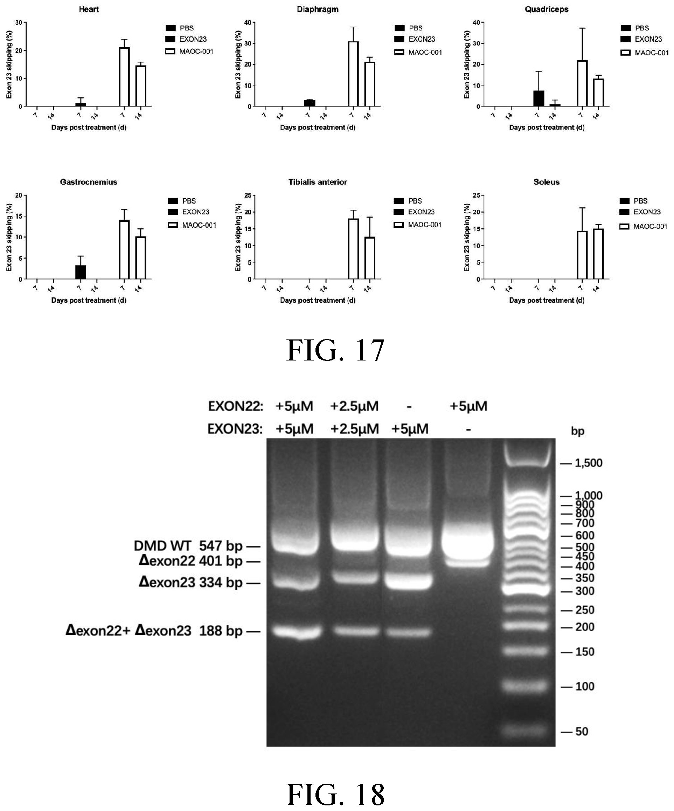

PCR reaction products were analyzed using a 3% TAE agarose gel. The product with wild-type (WT) DMD was 388 bp in length, while the product with DMD exon 23 skipping was 175 bp (Figure 16). The agarose gel electrophoresis results were analyzed and quantified using Image Lab software, and bar charts were generated using GraphPad Prism 9 software (Figure 17). The experimental results indicated that no exon skipping occurred in the PBS group; in the EXON23 PMO treatment group (G2), less than 10% exon 23 skipping was detected only in heart, diaphragm, quadriceps, and gastrocnemius tissues on day 7 post-treatment, and in quadriceps tissue on day 14 post-treatment; whereas in the MAOC-001 treatment group (G3), 10-30% exon 23 skipping was detected in all six sampled muscle tissues; the exon skipping efficacy of MAOC-001 was significantly better than that of EXON23 PMO.

### Example 12: In vitro DMD double-exon skipping assay

Mouse myoblasts C2C12 cells were seeded into a 12-well plate at 200,000 cells per well, and the plate was incubated overnight in an incubator at 37°C and 5% CO₂ using DMEM + 10% FBS as the culture medium. On the next day, the medium was replaced with differentiation medium of DMEM + 2% horse serum (Procell, Cat#164215) + 1 µM insulin (Procell, Cat#PB180432). The plate was placed in the incubator for further incubation for 3-5 days, with medium refreshed every 2 days. 10-fold concentrated EXON22 and EXON23 PMO were prepared, and 100 µL was added to 900 µL of fresh differentiation medium to incubate cells. The cells were harvested after 48 hours of drug treatment, and total RNA was extracted. The methods for total RNA extraction and exon skipping analysis were the same as in Example 11, and the primers used are listed in Table 6:

**Table 6: Dual-Exon Skipping Primers**

| | | |
|---|---|---|
| Primary PCR | Ex20F2 | 5'-ACCCAGTCTACCACCCTATC-3' |
| | Ex25R | 5'-CTCTTTATCTTCTGCCCACCTT-3' |
| Nested PCR | Ex21F | 5'-TACAACTGAAAGAAAAGGGACAG-3' (SEQ ID NO: 373) |
| | Ex24R | 5'-GCAGGCCATTCCTCTTTCAG-3' |

The PCR products were analyzed using a 3% TAE agarose gel. The product with wild-type (WT) DMD was 547 bp in length; the product with DMD exon 22 skipping was 401 bp, the product with DMD exon 23 skipping was 334 bp, and the product with both DMD exons 22 and 23 skipping was 188 bp (Figure 18). Analysis of the experimental results showed that only exon 22 skipping band was observed in samples treated with only 5 µM EXON22 PMO; both the exon 23 skipping band and the double exon 22 and 23 skipping band were observed in samples treated with only 5 µM EXON23 PMO, but the proportion of the exon 23 skipping band was greater; exon 22 skipping, exon 23 skipping, and double exons 22 and 23 skipping were observed in samples treated simultaneously with 2.5 µM EXON22 and EXON23 PMO, and the proportion of the double exon skipping band was the largest.

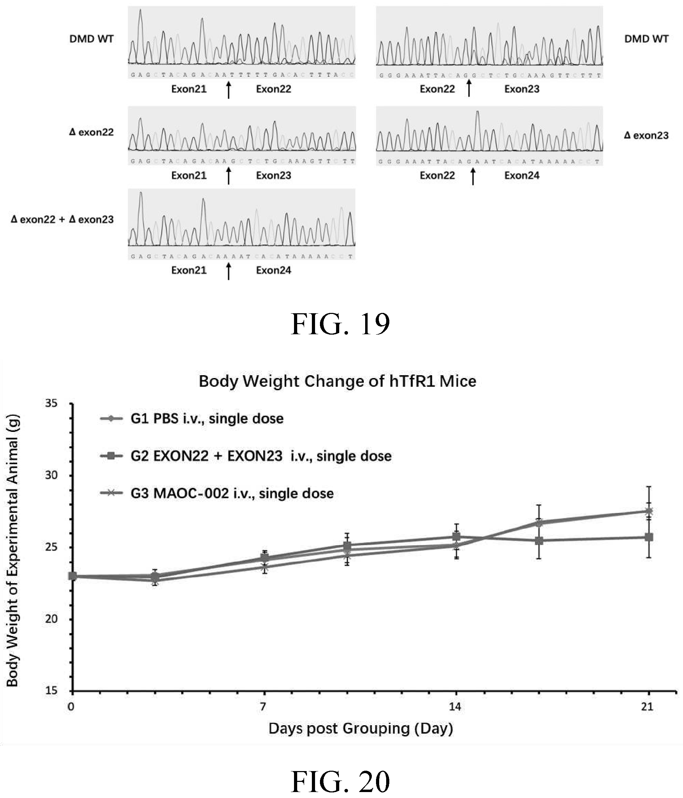

Four DNA fragments were separately recovered from the gel and sequenced to verify the correctness of the skipping sites (Figure 19), the sequencing results showed that all four sequences matched the sequences before and after the DMD skipping.

### Example 13: Analysis of the Delivery Efficiency of Two Oligonucleotides of MAOC-002 in Muscle Tissue of hTfR1 Humanized Mice

For the *in vivo* double-exon skipping experiment, hTfR1-humanized C57BL/6 mice weighing 19-25 g and aged 6-8 weeks were selected. Eighteen mice were grouped by body weight and randomly divided into three groups of six mice each. Each group received a single injection of the test drug at a dose of 600 nmol/kg, with a PBS group serving as the control. Tissue samples were collected at various time points to analyze the tissue distribution of drug in the mice and the *in vivo* double-exon skipping. Detailed administration information was shown in Table 7:

**Table 7-Grouping and Administration**

| **Group** | **Test Compound** | **Number of Animals (mice)** | **Dose (600 nmol/kg)** | **Administration Route** | **Administration Frequency ^{a}** |
|---|---|---|---|---|---|
| G1 | PBS | 6 | - | i.v. | Single dose |
| G2 | EXON22 + EXON23 | 6 | 600+600 | i.v. | Single dose |
| G3 | MAOC-002 | 6 | 600 | i.v. | Single dose |

| | | | | | |
|---|---|---|---|---|---|
| Note: a: A single dose refers to a single administration on the day of grouping; | | | | | |

All mice underwent clinical observation once daily, which included, but was not limited to, assessment of the animals' condition and feeding behavior. Body weight was measured twice weekly, and monitoring data showed that the body weights of all mice fluctuated within the normal range throughout the experiment, indicating that the mice tolerated the drug well at the tested doses (Figure 20). At the end of the experiment or upon reaching a humane endpoint, the animals were euthanized using an overdose of CO₂.

On days 4, 7, 14, and 21 after administration, three mice were selected from each group. After anesthesia, the mice underwent cardiac perfusion with pre-chilled PBS. After perfusion was completed, tissue samples of the heart, diaphragm, bilateral quadriceps, bilateral gastrocnemius, bilateral soleus, and bilateral tibialis anterior muscles were collected. After collection, each tissue sample was divided into two portions: the first portion was weighed, flash-frozen in liquid nitrogen, and stored at -80°C for analysis of PMO content in the tissue; the second portion was weighed and immersed in a solution of RNA Solid (Servicebio, Cat#G3019) with a volume 10 times that of the tissue; it was first incubated at 4°C overnight and then stored at -80°C for total RNA extraction and exon skipping analysis.

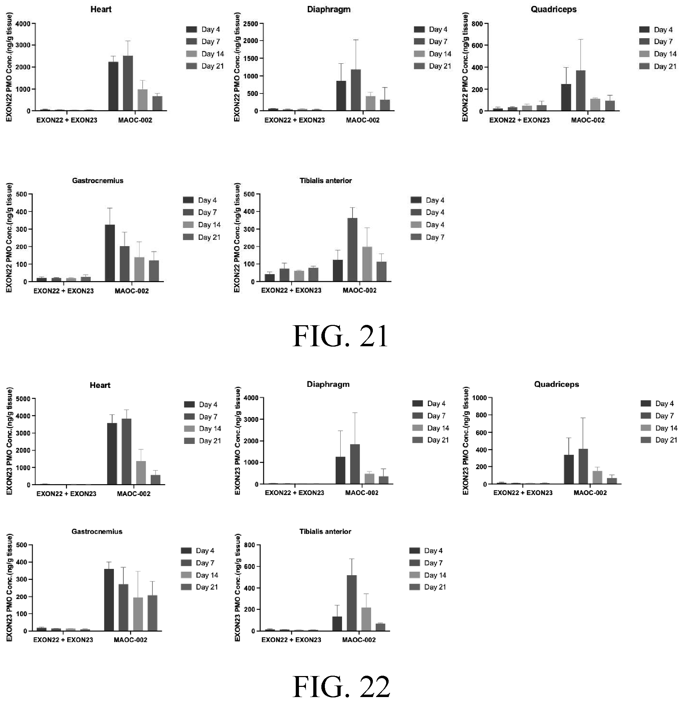

After the tissue samples were ground, the concentrations of EXON22 and EXON23 PMO in the tissues were measured separately using the ELISA method, following the same procedure as in Example 9. Bar charts were plotted based on the experimental data (Figures. 21 and 22). The experimental results showed that in all tissue samples, including the heart, diaphragm, quadriceps, gastrocnemius, and tibialis anterior, the EXON22 and EXON23 PMO levels in the MAOC-002 treatment group (G3) were significantly higher than those in the combined EXON22+EXON23 PMO treatment group (G2).

### Example 14: Analysis of the DMD double-exon skipping efficacy of MAOC-002 in hTfR1 humanized mice

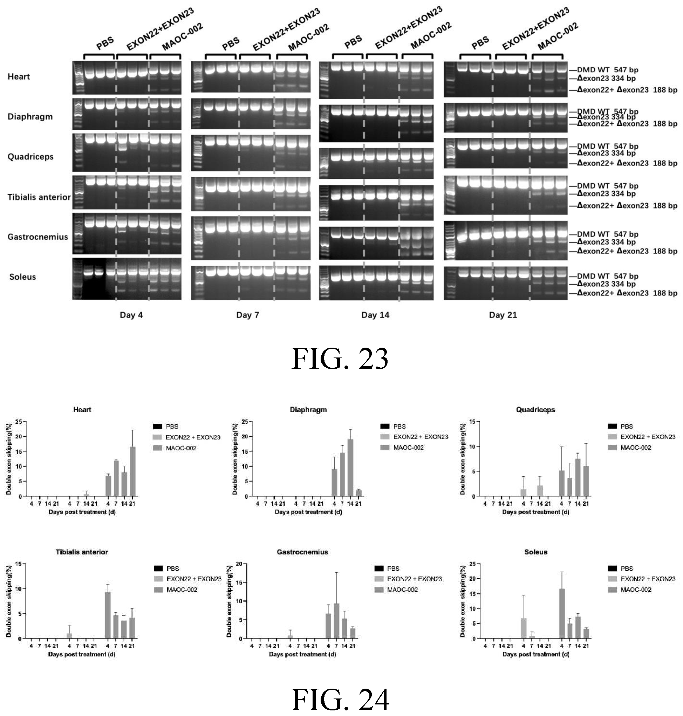

After each tissue sample was ground, total RNA was extracted and exon skipping analysis was performed using the same primers as in Example 12. PCR reaction products were analyzed using a 3% TAE agarose gel. The product with wild-type (WT) DMD was 547 bp in length, the product with DMD exon 22 skipping was 401 bp, the product with DMD exon 23 skipping DMD was 334 bp, and the product with DMD dual exon 22 and 23 skipping was 188 bp (Figure 23). The agarose gel electrophoresis results were analyzed and quantified using Image Lab software, and bar charts were generated using GraphPad Prism 9 software.

The experimental results were shown in Figure 24. No exon skipping occurred in the PBS group. In the combined EXON22 PMO and EXON23 PMO (1:1) treatment group (EXON22+EXON23, abbreviated as G2), dual exon skipping was essentially undetected in the six organ or tissue samples, only a small amount (about 5%) of dual exon skipping was detected in muscle tissue at individual time points after administration.

Surprisingly, the MAOC-002 treatment group (G3) in this study showed very significant double-exon skipping (about 10-20%) in all six sampled muscle tissues. This indicated that MAOC-002 successfully co-loaded EXON22 PMO and EXON23 PMO, and that MAOC-002, which co-loaded EXON22 PMO and EXON23 PMO, was significantly superior in its dual-exon skipping efficacy to the combined administration of EXON22 PMO and EXON23 PMO.

All documents cited in the present invention are incorporated by reference into this application, as if each were individually cited. Furthermore, it should be understood that, after reading the above description of the present invention, those skilled in the art may make various changes or modifications to the invention, and such equivalent forms are likewise within the scope defined by the appended claims.

## Claims

1. A polypeptide-oligonucleotide multimer complex, wherein the complex is a multimer formed by the complex of n monomers having complementary nucleic acid backbones, and n is a positive integer from 2 to 8;
wherein the monomer comprises:
(a) a polypeptide monomer, wherein the polypeptide monomer comprises: (a1) a polypeptide element and (a2) a first backbone nucleic acid single strand; and the polypeptide element is covalently linked to the first backbone nucleic acid single strand;
(b) an oligonucleotide monomer, wherein the oligonucleotide monomer comprises: (b1) an oligonucleotide pharmacodynamic element and (b2) a second backbone nucleic acid single strand; and the oligonucleotide pharmacodynamic element is covalently linked to the second backbone nucleic acid single strand; and
(c) an optional pure backbone monomer, wherein the pure backbone monomer comprises a third backbone nucleic acid single strand, and said third backbone nucleic acid single strand is not covalently linked to a targeting element (such as an antibody) nor to a pharmacodynamic element (such as an oligonucleotide drug);
and, in the multimer, the backbone nucleic acid single strand of each monomer forms a complementary double strand with the backbone nucleic acid single strands of other monomers through base pairing, thereby forming a complementary nucleic acid backbone composite structure;
and each complex carries at least one (a1) polypeptide element and at least one (b1) oligonucleotide pharmacodynamic element.

2. The polypeptide-oligonucleotide multimer complex according to claim 1, wherein the polypeptide element is selected from the group consisting of: a polypeptide element having a targeting function, a polypeptide element with an extended half-life, or a combination thereof.

3. The polypeptide-oligonucleotide multimer complex according to claim 1, wherein the polypeptide monomer has the structure of Formula I:
Z1-W1 (I)
wherein,
Z1 is a polypeptide moiety;
W1 is a first backbone nucleic acid single strand;
"-" is a linker or bond.

4. The polypeptide-oligonucleotide multimer complex according to claim 1, wherein the oligonucleotide monomer has the structure of Formula II:
Y1-W2-Y2 (II)
wherein,
Y1 is absent or a first oligonucleotide pharmacodynamic element;
Y2 is absent or a second oligonucleotide pharmacodynamic element;
W2 is a second backbone nucleic acid single strand;
"-" represents a linker or bond;
wherein Y1 and Y2 are not both absent, and the first oligonucleotide pharmacodynamic element and the second oligonucleotide pharmacodynamic element are the same or different.

5. The polypeptide-oligonucleotide multimer complex according to claim 1, wherein the pure backbone monomer has the structure of Formula III:
V1-W3-V2 (III)
wherein,
V1 is absent or a first detectable labeling group;
V2 is absent or a second detectable labeling group;
W3 is a third backbone nucleic acid single strand;
"-" is a linker or bond.

6. The polypeptide-oligonucleotide multimer complex according to claim 1, wherein the complex comprises the structure shown in Formula i:
(A-Y-)m-PNSC-(-Z-R)n (i)
wherein,
A comprises a polypeptide element having a targeting function;
R comprises an oligonucleotide pharmacodynamic element;
PNSC is a complementary nucleic acid backbone composite structure;
Y is a bond or a first linker;
Z is a bond or a second linker; and
m and n are each independently a positive integer of ≥1.

7. The polypeptide-oligonucleotide multimer complex according to claim 6, wherein the PNSC comprises the structure shown in Formula 1:
C1-R1-C2-R2-C3 (1)
wherein,
R1 is complementary base pairing region 1;
R2 is complementary base pairing region 2;
C1, C2, and C3 are each, independently, absent or a redundant nucleic acid;
"-" represents a bond.

8. A monomer library, wherein the monomer library comprises:
(a) a first monomer, the first monomer is a polypeptide monomer comprising: (a1) a polypeptide element and (a2) a first backbone nucleic acid single strand; and the polypeptide element is covalently linked to the first backbone nucleic acid single strand;
(b) a second monomer, the second monomer is an oligonucleotide monomer comprising: (b1) an oligonucleotide pharmacodynamic element and (b2) a second backbone nucleic acid single strand; and the oligonucleotide pharmacodynamic element is covalently linked to the second backbone nucleic acid single strand; and
(c) a third monomer, wherein the third monomer is a pure backbone monomer comprising a third backbone nucleic acid single strand, and the third backbone nucleic acid single strand is not covalently linked to a targeting element (such as an antibody) nor to a pharmacodynamic element (such as an oligonucleotide drug).

9. A pharmaceutical composition, wherein the pharmaceutical composition comprises:
(i) the polypeptide-oligonucleotide multimer complex according to claim 1 as an active ingredient; and
(ii) a pharmaceutically acceptable carrier.

10. A use of the polypeptide-oligonucleotide multimer complex according to claim 1 in the preparation of a medicament for treating a disease caused by abnormal gene expression.

11. A single-stranded nucleic acid sequence set for forming a hexameric complementary nucleic acid backbone composite structure, wherein the single-stranded nucleic acid sequence set is selected from the backbone nucleic acid single strand sequence sets listed in Table B under numbers 6-1 to 6-20.
